# EUROPEAN PATENT APPLICATION

(11) **EP 4 740 842 A1**
(43) Date of publication of application: **13.05.2026**
(21) Application number: 24861564.3
(22) Date of filing: 28.05.2024
(51) Int. Cl.: A61B 5/00

(54) **RESTING METABOLIC RATE MEASUREMENT METHOD, SYSTEM, AND MEDIUM**

(30) Priority: 07.09.2023 CN 202311156639
(71) Applicant: Huawei Technologies Co., Ltd., Shenzhen, Guangdong 518129 (CN)
(72) Inventor: YUAN, Jili, Shenzhen, Guangdong 518129 (CN); ZHAO, Shuai, Shenzhen, Guangdong 518129 (CN); REN, Huichao, Shenzhen, Guangdong 518129 (CN); CAO, Yu, Shenzhen, Guangdong 518129 (CN); QI, Biao, Shenzhen, Guangdong 518129 (CN)
(74) Representative: Thun, Clemens
(86) International application number: PCT/CN2024/095806
(87) International publication number: WO 2025/050716

(57) **Abstract**

Provided are a resting metabolic rate detection method and system, and a medium, to detect a resting metabolic rate more accurately by using a wearable device. In the method, the wearable device can collect at least one parameter among exercise data, a physiological parameter, a psychological parameter, and a sleep parameter, so that a more accurate resting metabolic rate can be obtained through detection based on impact of the collected parameter on the resting metabolic rate. In addition, a user interface can be further displayed based on the resting metabolic rate obtained through detection, and a user can be notified of change information about the resting metabolic rate through the user interface, so that the user can be guided to adjust an exercise plan, a dietary plan, a fat loss plan, or the like, thereby implementing more accurate and timely adjustment.

## Description

### CROSS-REFERENCE TO RELATED APPLICATIONS

This application claims priority to Chinese Patent Application No. 202311156639.4, filed with the China National Intellectual Property Administration on September 7, 2023 and entitled "RESTING METABOLIC RATE DETECTION METHOD AND SYSTEM, AND MEDIUM", which is incorporated herein by reference in its entirety.

### TECHNICAL FIELD

Embodiments of this application relate to the field of smart life technologies, and in particular, to a resting metabolic rate detection method and system, and a medium.

### BACKGROUND

A resting metabolic rate is an amount of energy consumed by a body at rest to sustain daily basic physiological activities (such as heartbeat, breathing, glandular secretion, blood circulation, and body temperature maintenance). The resting metabolic rate accounts for the highest proportion of total daily energy consumption of the body, reaching 60% to 75%. The resting metabolic rate is associated with factors including weight, body composition, gender, age, height, and the like, and is also affected by physical activity levels, exercise, dietary conditions (such as dieting and starvation), disease, medication, ambient temperature, genetic predisposition, and the like. Therefore, the resting metabolic rate actually changes dynamically.

How to implement dynamic detection on the resting metabolic rate holds important research significance.

### SUMMARY

Embodiments of this application provide a resting metabolic rate detection method and system, and a medium, to detect a resting metabolic rate more accurately by using a wearable device, so that a change of the resting metabolic rate can be assessed in time, and a user can more accurately know the resting metabolic rate of a body and then perform corresponding adjustment.

According to a first aspect, an embodiment of this application provides a resting metabolic rate detection method. The method includes the following steps: A wearable device collects at least one parameter among exercise data, a physiological parameter, a psychological parameter, and a sleep parameter of a user; the wearable device performs detection based on the at least one parameter, to obtain a resting metabolic rate; and the wearable device displays a first user interface based on the resting metabolic rate, where the first user interface is configured to indicate change information of the resting metabolic rate.

In the method, the wearable device collects various types of parameters in various scenarios, implementing assessment of a change of the resting metabolic rate from a plurality of aspects, so that a more accurate resting metabolic rate can be obtained through detection. In addition, a user interface can be displayed based on the dynamically changing resting metabolic rate obtained through detection, so that the user can be guided, through the user interface, to adjust an exercise plan, a dietary plan, a fat loss plan, or the like, thereby implementing more accurate and timely adjustment of a physical status.

In a possible implementation, the wearable device collects the exercise data of the user; and that the wearable device performs detection based on the exercise data, to obtain the resting metabolic rate includes: determining exercise duration and an average heart rate during exercise based on the exercise data; determining, based on the exercise duration, the average heart rate, and a preset matching relationship, exercise intensity that matches the exercise data, where the preset matching relationship indicates an exercise duration range and an average heart rate range that match different exercise intensity; and determining the resting metabolic rate based on the exercise intensity and the exercise duration, where the resting metabolic rate indicates a resting metabolic rate after the exercise.

In this implementation, the wearable device may more accurately detect the resting metabolic rate after the exercise based on the collected exercise data. In this way, based on more accurate data of the resting metabolic rate, the user can be guided more accurately for a scenario such as an exercise plan or a fat loss plan. For example, the user may be alerted to additional exercise to increase the resting metabolic rate, thereby promoting achievement of a calorie deficit, and achieving objectives of fat loss and the like. In another example, the user may be alerted to exercise reduction to avoid physical injury and the like. Compared with determining a resting metabolic rate only based on basic information such as age, gender, height, and weight, this scenario can implement more accurate detection of a resting metabolic rate, and can implement more accurate identification of an increase of a resting metabolic rate after exercise.

In a possible implementation, determining the resting metabolic rate based on the exercise intensity and the exercise duration includes: determining incremental information of the resting metabolic rate by using a pre-trained multi-parameter regression model with the exercise intensity and the exercise duration as inputs; and determining the resting metabolic rate after the exercise based on a resting metabolic rate before the exercise and the incremental information.

In this implementation, in consideration of impact of the exercise intensity and the exercise duration on the resting metabolic rate, the resting metabolic rate may be detected based on the exercise intensity and the exercise duration. For example, higher exercise intensity indicates a larger increment of the resting metabolic rate, and longer exercise duration indicates a larger increment of the resting metabolic rate. In this way, an actual resting metabolic rate after exercise can be detected in time, to better guide the user to adjust an exercise plan, a dietary plan, a fat loss plan, or the like.

In a possible implementation, the exercise data includes at least one of a PPG signal, an ACC signal, and an IMU. In this implementation, the exercise data can be collected more accurately by using various sensors included in the wearable device, so that a more accurate resting metabolic rate can be obtained through detection.

In a possible implementation, the first user interface includes an hourly, daily, weekly, monthly, or yearly resting metabolic rate and change information thereof, where in a case that the first user interface includes the hourly resting metabolic rate, when the wearable device collects the exercise data, the resting metabolic rate is higher than a resting metabolic rate obtained before the exercise data is collected. In this implementation, the interface showing a change of the resting metabolic rate may be displayed to the user based on the dynamically changing resting metabolic rate obtained through detection, so that the user can obtain the change of the resting metabolic rate more visually. For example, an hourly resting metabolic rate may be displayed, so that the user can check a change between a resting metabolic rate before exercise and a resting metabolic rate after exercise. In another example, a daily or weekly resting metabolic rate is displayed, so that the user can check a long-term trend of changes of the resting metabolic rate, and therefore the user can be guided to know the change of the resting metabolic rate in a more timely and accurate manner.

In a possible implementation, the first user interface includes at least one of daily calorie expenditure, daily calorie intake, and a daily calorie deficit, and the daily calorie deficit is determined based on a difference between the daily calorie expenditure and the daily calorie intake. In this implementation, more accurate daily calorie expenditure and daily calorie deficit may be obtained based on detection of the resting metabolic rate, so that the user can be guided more accurately for an exercise plan, a fat loss plan, a dietary plan, or the like.

In a possible implementation, before detection is performed to obtain the resting metabolic rate, the method further includes the following steps: the wearable device displays a second user interface, where the second user interface is configured to guide the user to input body composition information, and the second user interface includes a first control and a second control; and the wearable device obtains, in response to a first operation on the first control, the body composition information measured by a body fat scale; or the wearable device displays, in response to a second operation on the second control, at least one input interface, where the at least one input interface is configured to manually input at least one body composition parameter included in the body composition information; and that the wearable device performs detection based on the at least one parameter, to obtain the resting metabolic rate includes that: the wearable device performs detection based on the at least one parameter and the body composition parameter, to obtain the resting metabolic rate.

In this implementation, body composition data may be further collected by using the body fat scale, so that change trends of daily and hourly resting metabolic rates of the user can be evaluated based on parameter features of collected multi-source parameters. In this way, timely alerts for abnormal decreases and increases of the resting metabolic rate can be made based on the change trend of the resting metabolic rate, so that the user can be guided to maintain a healthy lifestyle. Compared with determining a resting metabolic rate only based on information such as age, gender, height, and weight, where the determined resting metabolic rate is fixed, this scenario can implement detection of a change trend of the resting metabolic rate, and can further provide guidance for a physical status based on the change trend of the resting metabolic rate.

In a possible implementation, after detection is performed to obtain the resting metabolic rate, the method further includes: displaying a third user interface based on the resting metabolic rate and a historical resting metabolic rate when a decrease of the resting metabolic rate meets a preset condition, where the third user interface is configured to alert the user to the decrease of the resting metabolic rate. For example, the wearable device stores daily calorie intake of the user, and when the daily calorie intake is less than a calorie intake threshold, the third user interface is further configured to make an alert for an excessive dieting state. In another example, when the wearable device detects that there is an ongoing fat loss plan, the third user interface is further configured to make an alert for a fat loss plateau.

In this implementation, long-term change trends of physiological, weight, body composition, and diet records are tracked and analyzed. Metabolic adaptation can be further identified based on a degree of a rate change and a change trend of the resting metabolic rate. Therefore, an excessive dieting state can be detected, and a fat loss plateau can be predicted. In this way, healthier and more scientific diet and exercise guidance can be provided, thereby implementing scientific fat loss and avoiding body injury.

In a possible implementation, after detection is performed to obtain the resting metabolic rate, the method further includes: sending the resting metabolic rate to a terminal device, where the terminal device is configured to display a fourth user interface based on the resting metabolic rate, and the fourth user interface is configured to indicate the change information of the resting metabolic rate.

In this implementation, the wearable device may further synchronize the resting metabolic rate to the terminal device (such as a mobile phone or a tablet computer). In this way, the user can check information about the resting metabolic rate on different devices.

In a possible implementation, the physiological parameter includes at least one of the following parameters: a resting heart rate, resting heart rate variability, a body temperature, a skin temperature, and a respiratory rate.

In this implementation, by collecting the physiological parameter, the wearable device can obtain a more accurate resting metabolic rate through detection in consideration of impact of different physiological parameters on the resting metabolic rate.

In a possible implementation, the psychological parameter includes at least one of the following parameters: a stress score and a stress level. In this implementation, by collecting the psychological parameter, the wearable device can obtain a more accurate resting metabolic rate through detection in consideration of impact of different psychological parameters on the resting metabolic rate.

In a possible implementation, the sleep parameter includes at least one of the following parameters: sleep time, sleep duration, sleep quality, and deep sleep continuity. In this implementation, by collecting the sleep parameter, the wearable device can obtain a more accurate resting metabolic rate through detection in consideration of impact of different sleep parameters on the resting metabolic rate.

In a possible implementation, that the wearable device displays the first user interface based on the resting metabolic rate includes that: the wearable device displays the first user interface based on the resting metabolic rate and the at least one parameter, where the first user interface is further configured to indicate a parameter that affects the change information of the resting metabolic rate.

In this implementation, a factor that affects a change of the resting metabolic rate may be further determined based on change information of the resting metabolic rate and change information of a parameter, and then the user may be alerted to the factor through the user interface, so that the user can more accurately know a trend and cause of the change of the resting metabolic rate. In this way, the user can be guided to more accurately adjust an exercise plan, a fat loss plan, a dietary plan, or the like.

According to a second aspect, an embodiment of this application further provides a wearable device, including one or more processors and one or more memories, where the one or more memories are configured to store one or more computer programs and data information, where the one or more computer programs include instructions; and when the instructions are executed by the one or more processors, the wearable device is enabled to perform the method in any possible design in the first aspect.

According to a third aspect, an embodiment of this application further provides a resting metabolic rate detection system, including the wearable device according to the second aspect and a terminal device (such as a mobile phone or a tablet computer). In addition, the wearable device may also send at least one collected parameter among exercise data, a physiological parameter, a psychological parameter, and a sleep parameter to the terminal device, for the terminal device to perform calculation to obtain a resting metabolic rate. In this way, a requirement on computing power of the wearable device can be reduced.

In a possible design, the system further includes a body fat scale.

According to a fourth aspect, an embodiment of this application provides a computer-readable storage medium. The computer-readable medium stores a computer program (which may also be referred to as code or instructions). When the computer program is run on a computer, the computer is enabled to perform the method in any possible implementation in the first aspect.

According to a fifth aspect, an embodiment of this application provides a computer program product. The computer program product includes a computer program (which may also be referred to as code or instructions). When the computer program is run, a computer is enabled to perform the method in any possible implementation in the first aspect.

According to a sixth aspect, an embodiment of this application further provides a graphical user interface on a wearable device. The wearable device has a display, one or more memories, and one or more processors. The one or more processors are configured to execute one or more computer programs stored in the one or more memories. The graphical user interface includes a graphical user interface displayed when the wearable device performs any possible implementation in the first aspect of embodiments of this application. The wearable device may be, for example, the wearable device included in the first aspect.

For beneficial effects of any one of the second aspect to the sixth aspect, specifically refer to beneficial effects of various possible designs in the first aspect. Details are not described herein again.

### BRIEF DESCRIPTION OF DRAWINGS

FIG. 1 is a diagram of a possible hardware structure of a terminal device according to an embodiment of this application;
FIG. 2 is a block diagram of a software architecture of a terminal device according to an embodiment of this application;
FIG. 3 is a schematic flowchart of resting metabolic rate detection according to an embodiment of this application;
FIG. 4A and FIG. 4B are a diagram 1 of interfaces for a resting metabolic rate detection method according to an embodiment of this application;
FIG. 5A, FIG. 5B, FIG. 5C, and FIG. 5D are a diagram 2 of interfaces for a resting metabolic rate detection method according to an embodiment of this application;
FIG. 6 is another schematic flowchart of a resting metabolic rate detection method according to an embodiment of this application;
FIG. 7A, FIG. 7B, FIG. 7C, FIG. 7D, and FIG. 7E are a diagram of interfaces for obtaining body composition information according to an embodiment of this application;
FIG. 8A, FIG. 8B, FIG. 8C, and FIG. 8D are a diagram 3 of interfaces for a resting metabolic rate detection method according to an embodiment of this application;
FIG. 9A, FIG. 9B, and FIG. 9C are a diagram 4 of interfaces for a resting metabolic rate detection method according to an embodiment of this application;
FIG. 10 is a diagram of a weight change rate according to an embodiment of this application;
FIG. 11 is still another schematic flowchart of a resting metabolic rate detection method according to an embodiment of this application;
FIG. 12A, FIG. 12B, and FIG. 12C are a diagram 5 of interfaces for a resting metabolic rate detection method according to an embodiment of this application; and
FIG. 13 is still another schematic flowchart of a resting metabolic rate detection method according to an embodiment of this application.

### DESCRIPTION OF EMBODIMENTS

The following describes some terms in this application, to facilitate understanding of a person skilled in the art.
(1) A resting metabolic rate is an amount of energy consumed by a body at rest to sustain daily basic physiological activities (such as heartbeat, breathing, glandular secretion, blood circulation, and body temperature maintenance). The resting metabolic rate accounts for the highest proportion of total daily energy consumption of the body, reaching 60% to 75%. The resting metabolic rate is associated with factors including weight, body composition, gender, age, height, and the like, and is also affected by physical activity levels, exercise, dietary conditions (such as dieting and starvation), disease, medication, ambient temperature, genetic predisposition, and the like.

A manner of measuring the resting metabolic rate is based on the principle of direct or indirect calorimetry. The direct calorimetry involves measuring heat that is released from a body and that is absorbed by water flowing in an internal pipe in an insulated chamber, to detect the resting metabolic rate. The indirect calorimetry involves measuring O₂ and CO₂ in respiratory gases by using an electrochemical or electromagnetic sensor with a wearable mask or hood, to calculate the resting metabolic rate. However, the direct or indirect calorimetry requires medium- and large-sized devices (such as a calorimetric chamber and a metabolic analyzer) for measurement. Consequently, limited by site requirements, measurement cannot be performed at any time. In addition, devices and instruments are expensive, resulting in high measurement costs.

Another manner of measuring the resting metabolic rate is based on basic information. Resting metabolic rates or resting calories are calculated based on the height, weight, age, and gender that are input by a user. However, the resting metabolic rate measured according to this method changes only with changes in the height, weight, age, and gender, and is substantially fixed. Consequently, an actual resting metabolic rate cannot be accurately reflected.

(2) A calorie deficit is a calorie deficit value obtained by subtracting daily total calorie intake from daily total calorie expenditure, where the daily total calorie intake is less than the daily total calorie expenditure. Usually, to achieve calorie balance, fat stored in a body is consumed through oxidation to fill the calorie deficit, thereby achieving weight loss or fat loss.

The calorie intake can be obtained through diet. The body's daily calorie expenditure may include three parts: basal metabolism (or resting metabolic rate, or resting metabolism for short), expenditure through daily activity and exercise, and thermic effect of food.

It may be understood that the resting metabolic rate is an important reference indicator for people who are seeking weight loss or fat loss. An important way to increase calorie expenditure is exercise. Besides expenditure during exercise, the resting metabolic rate of the body also increases within a period of time after the exercise, promoting achievement of the calorie deficit. In daily life, the resting metabolic rate changes with the fluctuation of factors such as physiological, psychological, and sleep statuses of the body.

(3) Metabolic adaptation is spontaneous reduction of calorie expenditure during weight loss. When the weight is significantly reduced, the body spontaneously performs adjustment to reduce a resting metabolic rate by an amount that is greater than a decrease of a resting metabolic rate caused by a weight change.

In view of this, embodiments of this application provide a resting metabolic rate detection method. In the method, resting metabolic rates of a user across different time scales can be assessed based on at least one of physiological, psychological, sleep, and exercise parameters collected by a wearable device such as a watch or a band, so that dynamic detection can be implemented on the resting metabolic rate. In addition, through the collection of the exercise parameter, an increase of the resting metabolic rate after exercise can be further detected. Moreover, a body composition parameter can be further collected by using a device, for example, a body fat scale, so that a change of the resting metabolic rate of the user can be more comprehensively assessed further with reference to the body composition parameter.

The assessment of the change of the resting metabolic rate may be applied to a plurality of application scenarios. For example, the application scenarios may include but are not limited to: customizing a weight loss or fat loss plan, adjusting dietary calorie intake, adjusting a training plan, alerts for insufficient exercise or excessive exercise, alerts for diet (such as dieting and excessive calorie intake), and detecting metabolic adaptation. Therefore, accurate detection of the resting metabolic rate can guide users in time to adjust their statuses, holding significant research value for the users to know and adjust their statuses.

The following describes in detail embodiments of this application with reference to accompanying drawings.

A method provided in embodiments of this application may be applied to a terminal device. It may be understood that the terminal device in embodiments of this application may be a device that can collect body-related information of a user, for example, a wearable device (such as a band, a watch, a helmet, or an anklet). It may be understood that a specific type of the terminal device is not limited in embodiments of this application.

The method provided in embodiments of this application may be further applied to a resting metabolic rate detection system. The system may include a first terminal device and a second terminal device. The first terminal device may be a device that can collect body-related information of a user, for example, a wearable device (such as a band, a watch, a helmet, or an anklet). The second terminal device may be a device for processing, such as a mobile phone, a tablet computer, or a notebook computer. In addition, the resting metabolic rate detection system may further include a third terminal device. The third terminal device may be a device for measuring a body composition parameter, for example, a body fat scale. Alternatively, the system may further include a server device. The server device may be configured to detect a resting metabolic rate based on various collected data. It may be understood that a quantity and types of terminal devices included in the system are not limited in embodiments of this application.

An example of an embodiment of the terminal device to which embodiments of this application may be applied or the terminal device included in the system includes but is not limited to a portable terminal device carrying HarmonyOS^{®}, iOS^{®}, Android^{®}, Microsoft^{®}, or another operating system. Alternatively, the portable terminal device may be another portable terminal device, for example, a laptop computer (Laptop) with a touch-sensitive surface (for example, a touch panel).

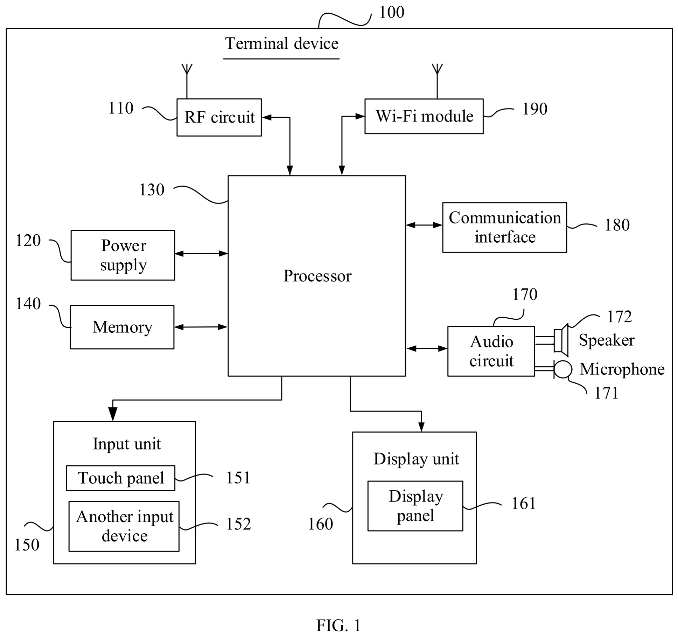

FIG. 1 is a diagram of a possible hardware structure of a terminal device. The terminal device 100 includes components such as a radio frequency (radio frequency, RF) circuit 110, a power supply 120, a processor 130, a memory 140, an input unit 150, a display unit 160, an audio circuit 170, a communication interface 180, and a wireless-fidelity (wireless-fidelity, Wi-Fi) module 190. A person skilled in the art may understand that the hardware structure of the terminal device 100 shown in FIG. 1 does not constitute a limitation on the terminal device 100. The terminal device 100 provided in embodiments of this application may include more or fewer components than those shown in the figure, or two or more components may be combined, or a different component configuration may be used. Various components shown in FIG. 1 may be implemented in hardware, software, or a combination of hardware and software that includes one or more signal processing and/or application-specific integrated circuits.

The components of the terminal device 100 are specifically described below with reference to FIG. 1.

The RF circuit 110 may be configured to receive and send data in a communication or call process. Specifically, after receiving downlink data from a base station, the RF circuit 110 sends the downlink data to the processor 130 for processing, and sends to-be-sent uplink data to the base station. The RF circuit 110 usually includes but is not limited to an antenna, at least one amplifier, a transceiver, a coupler, a low noise amplifier (low noise amplifier, LNA), and a duplexer.

In addition, the RF circuit 110 may also communicate with another device through a wireless communication network. The wireless communication may be implemented by using any communication standard or protocol, including but not limited to global system for mobile communications (global system for mobile communications, GSM), general packet radio service (general packet radio service, GPRS), code division multiple access (code division multiple access, CDMA), wideband code division multiple access (wideband code division multiple access, WCDMA), long term evolution (long term evolution, LTE), email, and short messaging service (short messaging service, SMS). In an embodiment of this application, for example, the terminal device 100 is a wearable device, and the wearable device may communicate with a mobile phone through the RF circuit 110.

A Wi-Fi technology is a short-range wireless transmission technology. The terminal device 100 may be connected to an access point (access point, AP) by the Wi-Fi module 190, to implement access to a data network. The Wi-Fi module 190 may be configured to receive and send data in a communication process. In an embodiment of this application, for example, the terminal device 100 is a wearable device, and may further communicate with a mobile phone through the Wi-Fi module 190. For example, the wearable device may synchronously transmit, through the Wi-Fi module 190, at least one of collected physiological, psychological, sleep, and exercise parameters to the mobile phone for display or for detecting a resting metabolic rate by the mobile phone.

The terminal device 100 may be physically connected to another device through the communication interface 180. Optionally, the communication interface 180 is connected to a communication interface of the another device by a cable, to implement data transmission between the terminal device 100 and the another device.

In embodiments of this application, the terminal device 100 can implement a communication service. For example, when the terminal device 100 is a wearable device, the terminal device 100 can interact with a mobile phone. Therefore, the terminal device 100 requires a data transmission function, that is, the terminal device 100 needs to include a communication module. Although FIG. 1 shows communication modules such as the RF circuit 110, the Wi-Fi module 190, and the communication interface 180, it may be understood that the terminal device 100 includes at least one of the foregoing components or another communication module (for example, a Bluetooth module) configured to implement communication, to perform data transmission.

For example, when the terminal device 100 is a wearable device, the terminal device 100 may include the RF circuit 110, and may further include the Wi-Fi module 190, or may include a Bluetooth module (not shown in FIG. 1). When the terminal device 100 is a mobile phone, the terminal device 100 may include the RF circuit 110, and may further include the Wi-Fi module 190, or may include a Bluetooth module (not shown in FIG. 1). In an embodiment of this application, the wearable device may also communicate with the mobile phone through the Bluetooth module.

The memory 140 may be configured to store a software program and a module. The processor 130 executes various function applications of the terminal device 100 and processes data by running the software program and the module that are stored in the memory 140. Optionally, the memory 140 may mainly include a program storage area and a data storage area. The program storage area may store an operating system (mainly including software programs or modules respectively corresponding to a kernel layer, a system layer, an application framework layer, an application layer, and the like). In addition, the memory 140 may include a high-speed random access memory, and may further include a non-volatile memory, for example, at least one magnetic disk storage device, a flash memory, or another volatile solid-state storage device. In an embodiment of this application, when the terminal device 100 is a wearable device, resting metabolic rates obtained through detection at different moments may be stored by the memory 140, to obtain a change trend of the resting metabolic rate within a specific time span, and the like.

The input unit 150 may be configured to: receive editing operations that are for different types of data objects such as digit or character information and that are input by a user, and generate a key signal input related to a user setting and function control of the terminal device 100. Optionally, the input unit 150 may include a touch panel 151 and another input device 152.

The touch panel 151, also referred to as a touchscreen, may collect a touch operation of a user on or near the touch panel 151 (for example, an operation of the user on or near the touch panel 151 by using any suitable object or accessory such as a finger or a stylus), and drive a corresponding connection apparatus based on a preset program.

Optionally, the another input device 152 may include but is not limited to one or more of a physical keyboard, a function button (such as a volume control button or a switch button), a trackball, a mouse, and a joystick.

The display unit 160 may be configured to display information input by a user or information provided for a user, and various menus of the terminal device 100. The display unit 160 is a display system of the terminal device 100, and is configured to present an interface to implement human-computer interaction. The display unit 160 may include a display panel 161. Optionally, the display panel 161 may be configured in a form of a liquid crystal display (liquid crystal display, LCD), an organic light-emitting diode (organic light-emitting diode, OLED), or the like. In an embodiment of this application, the display unit 160 may display a resting metabolic rate obtained through detection, and may further display a change trend of the resting metabolic rate in one day, one week, one month, or even one year, so that a user can track the change of the resting metabolic rate for a long time, and then adjust a status of the user in time.

The processor 130 is a control center of the terminal device 100 and is connected to the components by various interfaces and lines. The processor 130 runs or executes the software program and/or module stored in the memory 140, and calls the data stored in the memory 140, to perform various functions of the terminal device 100 and process data, to implement a plurality of services based on the terminal device 100. In an embodiment of this application, the processor 130 is configured to implement the method provided in embodiments of this application, to detect a resting metabolic rate more accurately, and track, guide, and adjust a physical status more comprehensively and accurately based on the resting metabolic rate obtained through detection.

The terminal device 100 further includes the power supply 120 (for example, a battery) configured to supply power to each component. Optionally, the power supply 120 may be logically connected to the processor 130 by a power management system, to implement functions such as charging, discharging, and power consumption by using the power management system.

As shown in FIG. 1, the terminal device 100 further includes the audio circuit 170, a microphone 171, and a speaker 172, and may provide an audio interface between the user and the terminal device 100. The audio circuit 170 may be configured to convert audio data into a signal that can be identified by the speaker 172, and transmit the signal to the speaker 172. The speaker 172 converts the signal into a sound signal for output. The microphone 171 is configured to collect an external sound signal (for example, a sound of a person speaking, or another sound), convert the collected external sound signal into a signal that can be identified by the audio circuit 170, and send the signal to the audio circuit 170. The audio circuit 170 may be further configured to convert the signal sent by the microphone 171 into audio data, and then output the audio data to the RF circuit 110 for sending to, for example, another terminal device, or output the audio data to the memory 140 for subsequent processing.

The terminal device 100 may further include at least one sensor, a camera, and the like, although not shown in the figure. Details are not described herein. The at least one sensor may include but is not limited to a pressure sensor, a barometric pressure sensor, an acceleration sensor, a distance sensor, a fingerprint sensor, a touch sensor, and a temperature sensor. In an embodiment of this application, if the terminal device 100 is a wearable device on a hand or foot, the terminal device 100 may include an acceleration sensor, and may monitor an exercise parameter and the like of the user by using the acceleration sensor; or may include a temperature sensor, and may monitor information such as a body temperature and a skin temperature of the user by using the temperature sensor.

An operating system (operating system, OS) in embodiments of this application is the most basic system software running on the terminal device 100. For example, an operating system of a mobile phone may be HarmonyOS (HarmonyOS), Android (Android), or iOS. A layered architecture, an event-driven architecture, a microkernel architecture, a microservice architecture, or a cloud architecture may be used for a software system of the terminal device 100. In an embodiment of this application, an operating system with a layered architecture is used as an example to describe a software architecture of the terminal device 100.

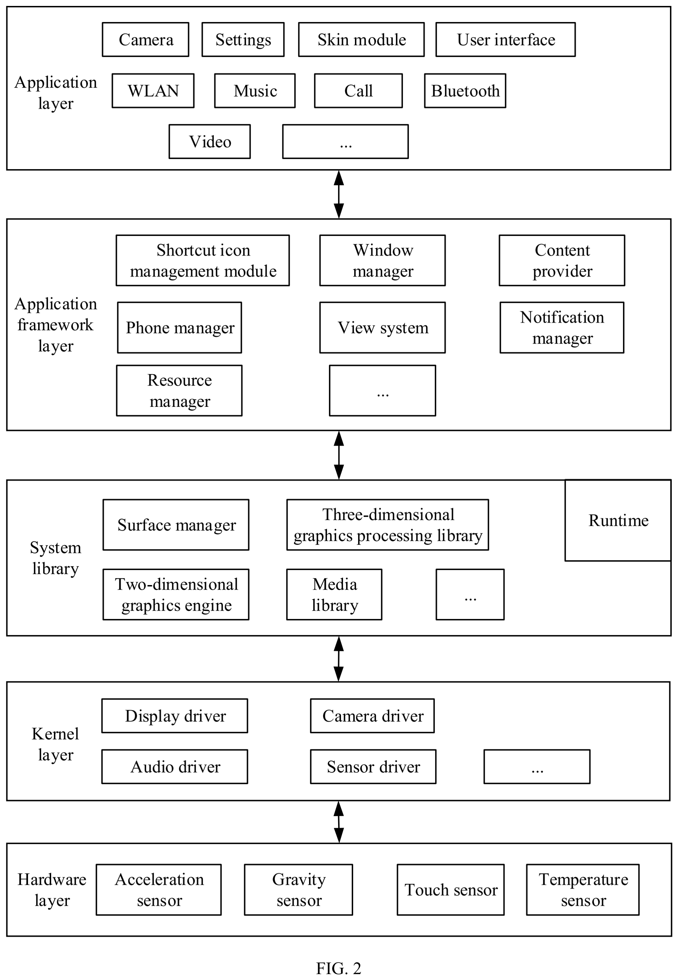

FIG. 2 is a block diagram of a software architecture of a terminal device according to an embodiment of this application. As shown in FIG. 2, the software architecture of the terminal device may be a layered architecture. For example, the software may be divided into several layers, and each layer has a clear role and task. The layers communicate with each other through a software interface. In some embodiments, an operating system is divided into five layers: an application layer, an application framework (framework, FWK) layer, runtime and a system library, a kernel layer, and a hardware layer from top to bottom.

The application layer may include a series of application packages. As shown in FIG. 2, the application layer may include Camera, Settings, a skin module, a user interface (user interface, UI), a third-party application, and the like. The third-party application may include WLAN, Music, Call, Bluetooth, Video, and the like.

In a possible implementation, an application may be developed by using Java language and completed by calling an application programming interface (application programming interface, API) provided by the application framework layer. A developer may interact with a bottom layer (such as the hardware layer or the kernel layer) of the operating system through the application framework layer, to develop an application of the developer. The application framework layer mainly includes a series of services and management systems of the operating system.

The application framework layer provides an application programming interface and a programming framework for an application at the application layer. The application framework layer includes some predefined functions. As shown in FIG. 2, the application framework layer may include a shortcut icon management module, a window manager, a content provider, a view system, a phone manager, a resource manager, a notification manager, and the like.

The shortcut icon management module is configured to manage a shortcut icon displayed on the terminal device, for example, create the shortcut icon, remove the shortcut icon, or monitor whether the shortcut icon meets a display condition.

The window manager is configured to manage a window program. The window manager may obtain a size of a display, determine whether there is a status bar, perform screen locking, take a screenshot, and the like. The content provider is configured to store and obtain data, and enable the data to be accessed by an application. The data may include a video, an image, audio, calls that are made and answered, a browsing history and bookmark, an address book, and the like.

The view system includes a visual control such as a control for displaying text or a control for displaying an image. The view system may be configured to construct an application. A display interface may include one or more views. For example, a display interface including a short-message notification icon may include a text display view and an image display view.

The phone manager is configured to provide a communication function of the terminal device, for example, management of a call status (including answered, ended, or the like). It may be understood that some wearable devices may not include a phone manager.

The resource manager provides various resources, such as a localized character string, an icon, an image, a layout file, and a video file, for an application.

The notification manager enables an application to display notification information in a status bar, and may be configured to convey a notification message. The notification message may automatically disappear after a short pause without requiring a user interaction. For example, the notification manager is configured to notify download completion, provide a message notification, and the like. The notification manager may also provide a notification that appears in a status bar at the top of the system in a form of a graph or a text scroll bar, for example, a notification of an application that is run on a background, or may provide a notification that appears on a screen in a form of a dialog window. For example, text information is displayed in the status bar, an alert tone is made, the terminal device vibrates, or an indicator light blinks.

In some embodiments of this application, the application framework layer is mainly responsible for calling a service interface for communication with the hardware layer, to transfer an operation request for a user to perform an operation to the hardware layer. The operation request may include checking, by the user, a resting metabolic rate through the display unit 160, and the like.

The runtime includes a core library and a virtual machine. The runtime is responsible for scheduling and managing the operating system.

The core library includes two parts: a functional function that needs to be called in Java language, and a core library of the operating system. The application layer and the application framework layer are run on the virtual machine. The virtual machine executes Java files of the application layer and the application framework layer as binary files. The virtual machine is configured to implement functions such as object lifecycle management, stack management, thread management, security and exception management, and garbage collection.

The system library may include a plurality of functional modules, for example, a surface manager (surface manager), a media library (media library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in various common audio and video formats, and static image files. The media library may support various audio and video coding formats, such as MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

In some embodiments, the three-dimensional graphics processing library may be configured to draw a three-dimensional moving image, and the 2D graphics engine may be configured to draw a two-dimensional moving image.

The kernel layer is a layer between hardware and software. The kernel layer includes at least a display driver, a camera driver, an audio driver, and a sensor driver.

The hardware layer may include various sensors, such as an acceleration sensor, a gravity sensor, a touch sensor, and a temperature sensor.

Usually, the terminal device 100 may run a plurality of applications simultaneously. In a simple case, one application may correspond to one process. In a complex case, one application may correspond to a plurality of processes. Each process has a process number (process ID).

With reference to the description of the hardware structure of the terminal device in FIG. 1 and the description of the software framework of the terminal device in FIG. 2, the following describes, by using examples with reference to a plurality of embodiments and accompanying drawings, operating principles of software and hardware for the terminal device to perform a resting metabolic rate detection method provided in embodiments of this application.

It should be understood that, in embodiments of this application, "at least one" means one or more, and "a plurality of" means two or more. "And/or" describes an association relationship between associated objects, and indicates that three relationships may exist. For example, A and/or B may indicate the following three cases: Only A exists, both A and B exist, and only B exists, where A and B may be singular or plural. The character "/" usually indicates an "or" relationship between the associated objects. "At least one of the following items (pieces)" or a similar expression thereof refers to any combination of these items, including any combination of singular items (pieces) or plural items (pieces). For example, at least one of a, b, or c may indicate: a, b, c, a and b, a and c, b and c, or a, b and c, where a, b, and c may be singular or plural.

"A plurality of" in embodiments of this application means "greater than or equal to two".

In addition, it should be understood that in description of this application, terms such as "first" and "second" are merely used for distinguishing and description, but should not be understood as indicating or implying relative importance, or should not be understood as indicating or implying a sequence.

In addition, in embodiments of this application, "terminal device", "device", and the like may be used interchangeably, that is, may be various devices that can be used to implement embodiments of this application.

It should be understood that the hardware structure of the terminal device may be shown in FIG. 1, and the software architecture of the terminal device may be shown in FIG. 2. The software program and/or module corresponding to the software architecture of the terminal device may be stored in the memory 140. The processor 130 may run the software program and application stored in the memory 140, to perform a procedure of a resting metabolic rate detection method provided in embodiments of this application.

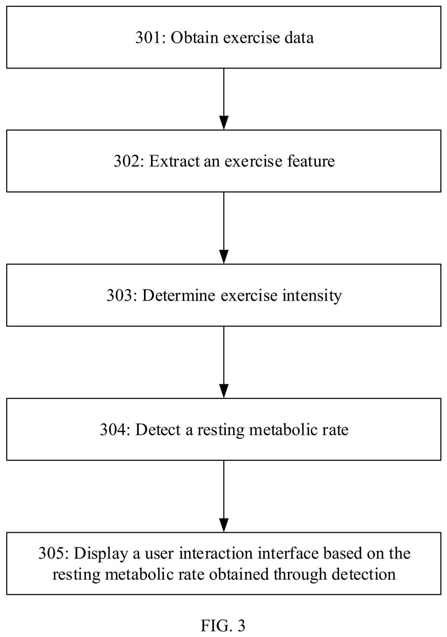

For ease of understanding of a resting metabolic rate detection method provided in this application, the following describes, with reference to content shown in FIG. 3 to FIG. 12C, an implementation process of the method provided in this application. In the following embodiments, the method provided in embodiments of this application may be described by using several possible scenarios. It may be understood that embodiments of this application are not limited to the following several scenarios.

Scenario A: Exercise scenario. Each time a user completes exercise of specific intensity, an increase of a resting metabolic rate of the user may be assessed in time, to provide positive feedback of fat loss and the like for the user. For example, FIG. 3 is a schematic flowchart of resting metabolic rate detection according to an embodiment of this application. The procedure may include the following steps.

Step 301: Obtain exercise data.

In an optional embodiment, a wearable device may detect an exercise type through manual input by the user. For example, the wearable device may detect an operation that is performed by the user on the wearable device and that is for triggering training, and determine an exercise type for the user to perform training, for example, aerobic exercise, anaerobic exercise, or mixed aerobic and anaerobic exercise (which may be understood as a combination of aerobic exercise and anaerobic exercise). In another example, a mobile phone may detect an operation that is performed by the user on the mobile phone and that is for triggering training. When the mobile phone and the wearable device are in a connected state, the wearable device may also determine the exercise type through the mobile phone.

In another optional embodiment, the wearable device may detect the exercise type through automatic detection. For example, the wearable device may detect, by using an acceleration sensor, a plurality of large-range and high-frequency hand or foot movements of the user in a short period of time, and may identify that the user is in an exercise state. For example, the wearable device may send a notification to the user, for the user to determine whether the user is in the exercise state, and for the wearable device to obtain the exercise type from the user. In another example, the wearable device may automatically identify the exercise type based on collected data and/or historical exercise data. In addition, the wearable device may further send a notification including an identification result to the user, to obtain feedback from the user, improving identification accuracy.

In a case that the wearable device detects that the user is in the exercise state, a photoplethysmography (photoplethysmography, PPG) signal, an accelerometer (accelerometer, ACC) signal, and an inertial measurement unit (inertial measurement unit, IMU) may be collected, but this is not limited thereto. The PPG signal, the ACC signal, the IMU signal, and the like may be used to extract exercise features.

Step 302: Extract an exercise feature.

In an optional embodiment, peaks and valleys during exercise may be extracted based on the collected PPG signal, to obtain a real-time heart rate sequence; and an average heart rate (hr_mean), a maximum heart rate (hr_max), and heart rate fluctuation (hr_cv) that correspond to the real-time heart rate sequence may be extracted after the exercise. In addition, maximum oxygen uptake and other information may be further inferred based on the real-time heart rate sequence.

In another optional embodiment, features that reflect intensity of movement, such as an average acceleration amplitude (acc_mean), a maximum acceleration amplitude (acc_max), and acceleration fluctuation (acc_cv), may be extracted based on the collected ACC signal and/or IMU signal.

Step 303: Determine exercise intensity.

In an optional embodiment, exercise intensity threshold conditions, such as an exercise duration threshold and an average heart rate threshold, may be set for different exercise types, to determine exercise intensity. For example, through classification, there may be primarily three exercise types, including aerobic exercise, anaerobic exercise, and mixed aerobic and anaerobic exercise. For aerobic exercise, a corresponding exercise intensity threshold condition may be set. For anaerobic exercise, a corresponding exercise intensity threshold condition may also be set. For mixed aerobic and anaerobic exercise, a corresponding exercise intensity threshold condition may also be set.

For example, an exercise type of running is aerobic exercise. For example, an exercise intensity threshold condition of aerobic exercise may be shown in Table 1:

**Table 1**

| | Initial condition (weak) | Moderate | Strong |
|---|---|---|---|
| Exercise duration | ≥ thresh_sd0 | ≥ thresh_sd1 | ≥ thresh_sd2 |
| Average heart rate | ≥ thresh_hr0 | ≥ thresh_hr1 | ≥ thresh_hr2 |

As shown in Table 1, the aerobic exercise may include weak exercise intensity, moderate exercise intensity, and strong exercise intensity. An initial condition of the exercise intensity may be further set. It is considered that the initial condition of exercise is met only when the exercise duration is greater than or equal to thresh _sd0 and the average heart rate is greater than or equal to thresh_hr0, and then the exercise intensity can be further determined. It may be understood that, when the initial condition is not met, the exercise intensity is too low to increase a resting metabolic rate after the exercise. Further, when the initial condition is met but a threshold condition under which the exercise intensity is moderate is not met, it can be determined that the exercise intensity is weak. Similarly, moderate or strong exercise intensity can be determined. The exercise intensity of specific exercise can be determined based on actually extracted exercise features: exercise duration and an average heart rate.

In addition, the exercise intensity may be further used as one basis for determining duration of an increase of the resting metabolic rate after exercise. It may be understood that stronger exercise intensity indicates longer duration of an increase of the resting metabolic rate.

Step 304: Detect a resting metabolic rate.

In an optional embodiment, a change amount of the resting metabolic rate after exercise may be related to exercise intensity and exercise duration during the exercise. It may be understood that stronger exercise intensity indicates longer duration of an increase of the resting metabolic rate; and longer exercise duration indicates longer duration of an increase of the resting metabolic rate.

In another optional embodiment, a change amount of the resting metabolic rate after exercise may be related to individual body quality. For example, maximum oxygen uptake may be further obtained to reflect a cardiopulmonary function and a muscle oxygen utilization capability in the individual body quality, and information, such as exercise frequency, exercise duration, and exercise type, stored in a wearable device or a connected mobile phone may be obtained to reflect historical physical activity levels. For example, a stronger cardiopulmonary function and a stronger muscle oxygen utilization capability usually indicate a higher resting metabolic rate level.

Based on the obtained exercise intensity, individual body quality, and historical physical activity levels, with a resting metabolic rate before exercise, a multi-parameter regression model is established by machine learning or another method, and an increment of a resting metabolic rate after the exercise is obtained, so that the resting metabolic rate after the exercise can be detected.

Step 305: Display a user interaction interface based on the resting metabolic rate obtained through detection.

The user interaction interface may be displayed on a wearable device (for example, a device such as a smartwatch or a smart band) or may be displayed on a mobile phone.

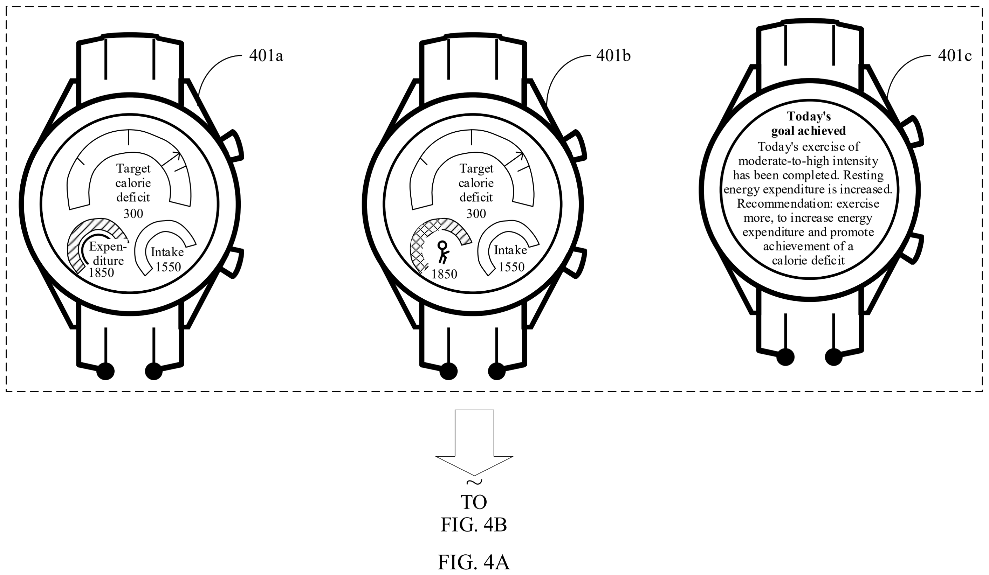

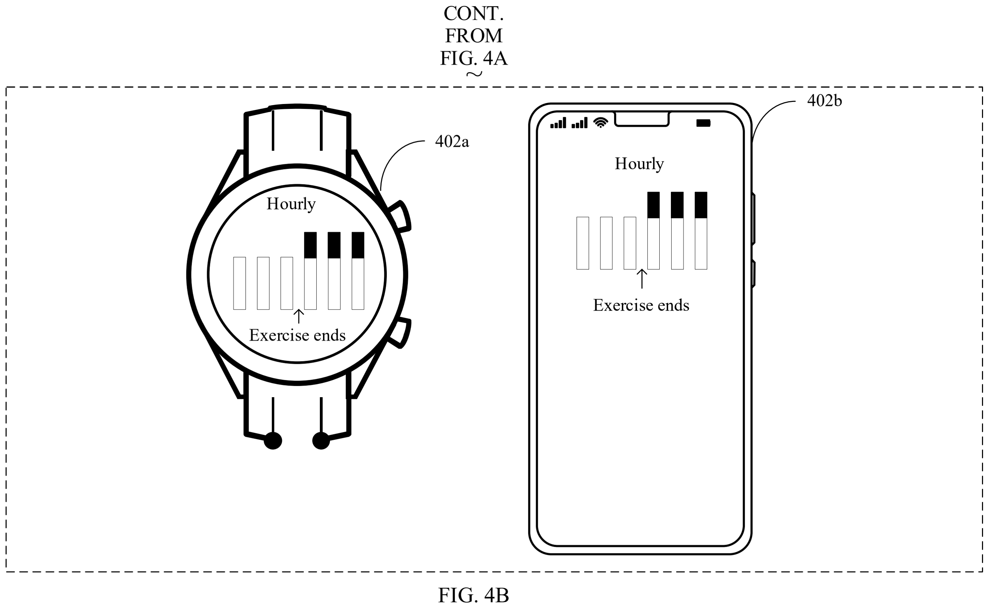

For example, FIG. 4A and FIG. 4B are a diagram of interfaces for a resting metabolic rate detection method according to an embodiment of this application. For example, the wearable device is a smartwatch, and the smartwatch may display an interface 401a or an interface 401b. For example, the interface 401a or the interface 401b may include daily calorie expenditure, daily calorie intake, a daily calorie deficit, and the like. It should be noted that a display form of information such as daily calorie expenditure, daily calorie intake, and a daily calorie deficit on the interface is not limited in embodiments of this application, for example, may be a graph, text, or chart form, and the interface 401a and the interface 401b are merely two possible examples.

In some possible scenarios, the smartwatch may further send a notification to the user in a form of a notification bar, vibration, a ringtone, a pop-up window, or the like after detecting that the user completes exercise of specific intensity today, for example, may display an interface 401c. The interface 401c may be configured to alert the user that a today's exercise goal has been achieved. In addition, an alert may be further sent to the user based on a daily calorie deficit. For example, the user may be alerted to exercise more to promote achievement of a daily calorie deficit.

In some possible embodiments, the smartwatch may further display an interface 402a in response to an operation on the interface 401a or the interface 401b or the interface 401c, for example, in response to a touch operation on daily calorie expenditure in the interface 401a. The interface 402a may be configured to display a change of an hourly resting metabolic rate, for example, may display an average resting metabolic rate per hour. As shown in the interface 402a, the resting metabolic rate significantly increases after exercise. In addition, the interface 402a may further display incremental information of the resting metabolic rate after the exercise, so that the user can visually check an increase of the resting metabolic rate after the exercise. For example, a black block part in the interface 402a represents the incremental information of the resting metabolic rate after the exercise.

In addition, it should be noted that, after the smartwatch establishes a connection to a device such as a mobile phone or a tablet computer, the smartwatch may further synchronize exercise data and the like to the device such as the mobile phone or the tablet computer. Therefore, the user can also check a change of the resting metabolic rate by using the mobile phone. For example, the mobile phone may display an interface 402b, and the interface 402b may be configured to display a change of an hourly resting metabolic rate.

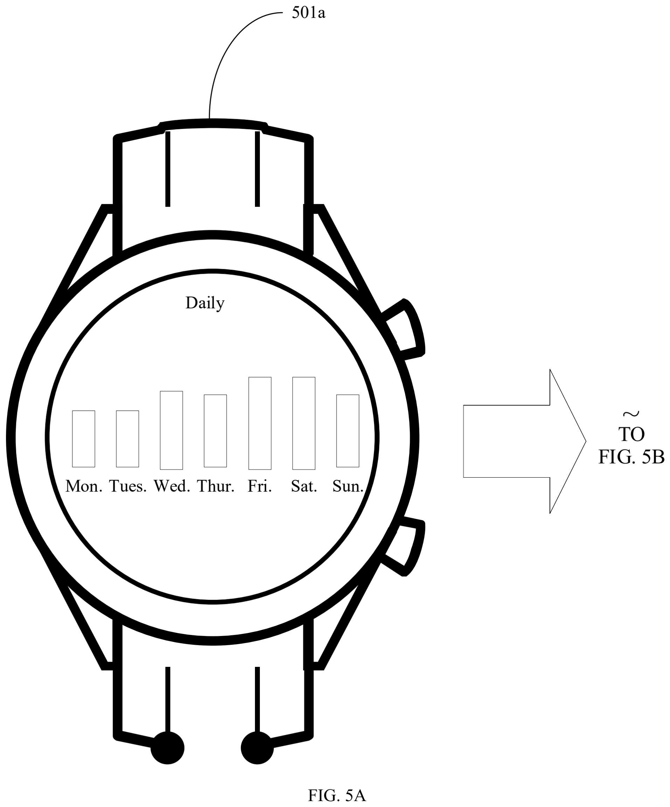

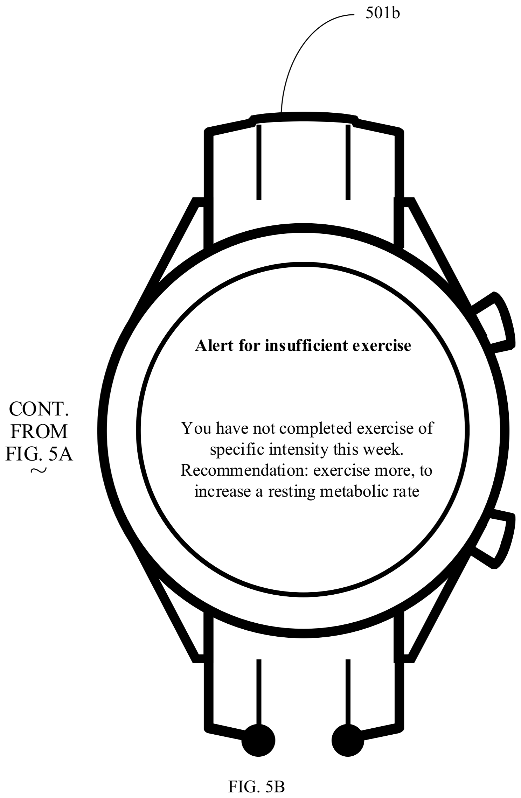

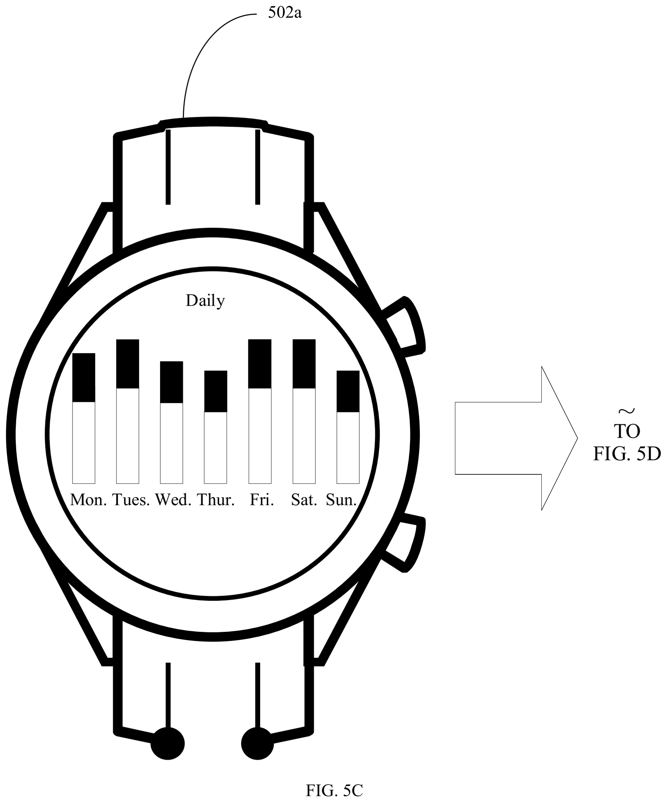

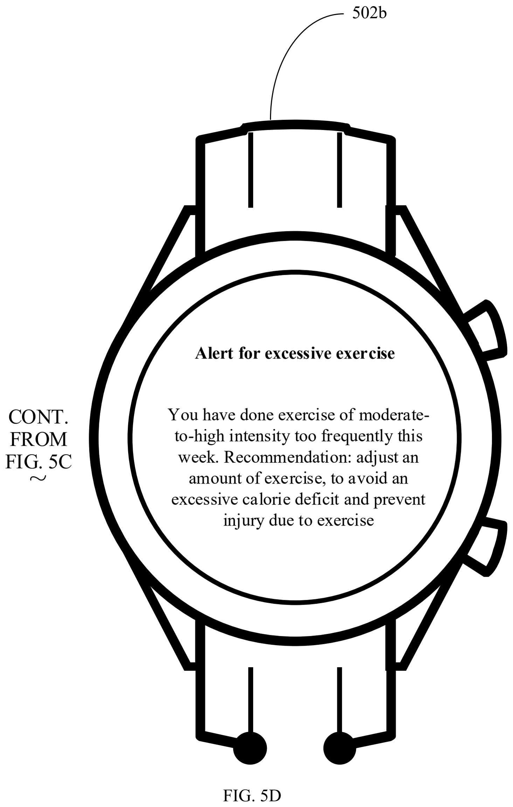

In some possible embodiments, the smartwatch, the mobile phone, or the like may further switch, in response to a user operation, to display a change of a daily resting metabolic rate. For example, FIG. 5A, FIG. 5B, FIG. 5C, and FIG. 5D are another diagram of interfaces for a resting metabolic rate detection method according to an embodiment of this application. For example, the smartwatch may display an interface 501a or an interface 502a shown in FIG. 5A or FIG. 5C in response to an operation (for example, a left-right pinch operation) on the interface 402a. The interface 501a and the interface 502a may be configured to display a change of daily average resting metabolic rates in a week. It should be noted that a display form of the change of the daily resting metabolic rate is not limited in embodiments of this application, for example, may be a form of a chart, a polyline graph, or a circular ring.

In some possible scenarios, the smartwatch may further analyze a change trend of the resting metabolic rate in a fixed cycle, and send a notification to the user based on an analysis result. The fixed cycle may be, for example, every three days, every week, every month, or every year. This is not limited in this application. An example in which the fixed cycle is every week is used. The smartwatch may display an interface 501b based on a change trend of the resting metabolic rate indicated on the interface 501a. The interface 501b may be configured to indicate an alert for insufficient exercise in this week. In another example, the smartwatch may display an interface 502b based on a change trend of the resting metabolic rate indicated on the interface 502a. The interface 502b may be configured to indicate an alert for excessive exercise in this week.

According to the content described in the scenario A, the wearable device can obtain exercise data, and more accurately detect a resting metabolic rate after exercise based on the exercise data. In this way, based on more accurate data of the resting metabolic rate, the user can be guided more accurately for a scenario such as an exercise plan or a fat loss plan. For example, the user may be alerted to additional exercise to increase the resting metabolic rate, thereby promoting achievement of a calorie deficit, and achieving objectives of fat loss and the like. In another example, the user may be alerted to exercise reduction to avoid physical injury and the like. Compared with determining a resting metabolic rate only based on basic information such as age, gender, height, and weight, this scenario can implement more accurate detection of a resting metabolic rate, and can implement more accurate identification of an increase of a resting metabolic rate after exercise.

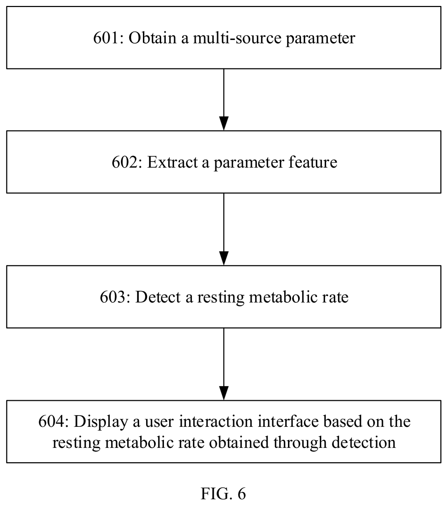

Scenario B: Daily life scenario. Fluctuation of a resting metabolic rate caused by changes of physiological, psychological, and sleep statuses of a user may be analyzed, to guide the user to adjust a status of the user and maintain a healthy lifestyle. In addition, a change of the resting metabolic rate may be more comprehensively analyzed further with reference to a change of a body composition parameter. For example, FIG. 6 is another schematic flowchart of a resting metabolic rate detection method according to an embodiment of this application. The procedure may include the following steps.

Step 601: Obtain a multi-source parameter.

In an optional embodiment, real-time physiological, psychological, and sleep statuses of the user may be obtained by using a wearable device. For example, the wearable device such as a smartwatch or a smart band may collect a physiological parameter, a psychological parameter, a sleep parameter, and the like by using a sensor such as a PPG sensor or a thermistor (negative temperature coefficient, NTC) temperature sensor. The physiological parameter may include but is not limited to a resting heart rate, resting heart rate variability, a body temperature, a skin temperature, and a respiratory rate. The psychological parameter may include but is not limited to a stress score and a stress level. The sleep parameter may include but is not limited to sleep time, sleep duration, sleep quality, and deep sleep continuity. The psychological parameter and the sleep parameter may be inferred from the physiological parameter. For example, the psychological parameter may be obtained based on the resting heart rate variability. It may be understood that the wearable device may synchronize the collected physiological, psychological, and sleep parameters to a connected mobile phone, or upload the collected physiological, psychological, and sleep parameters to a cloud server, or the like.

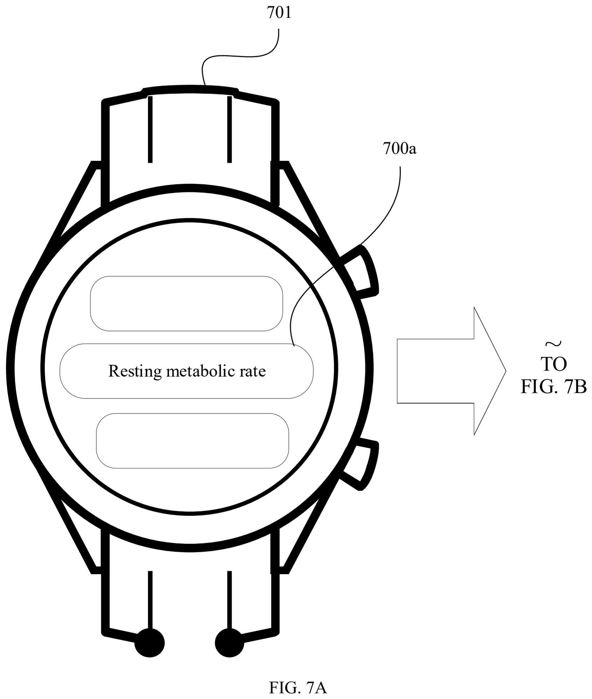

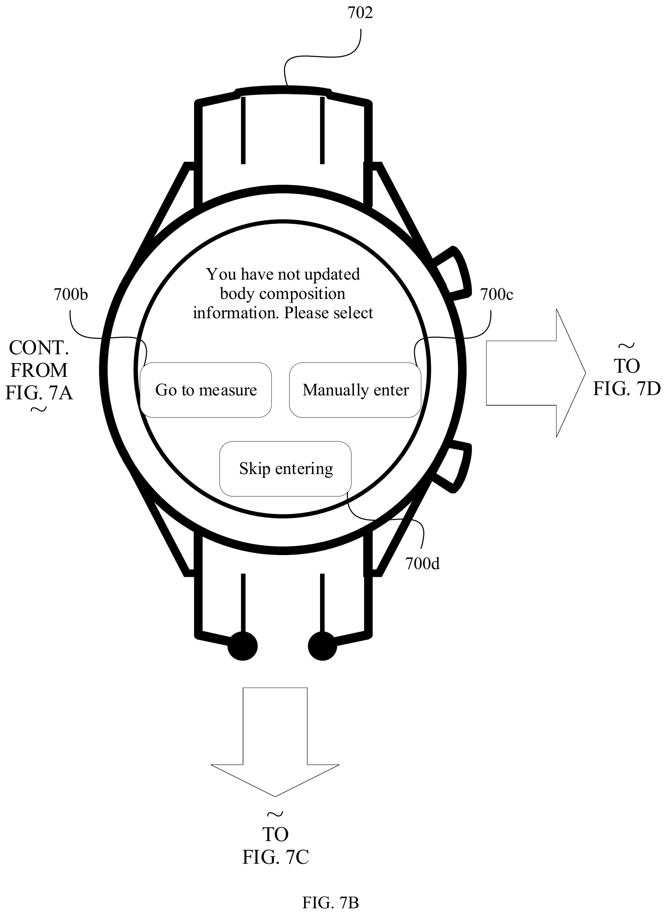

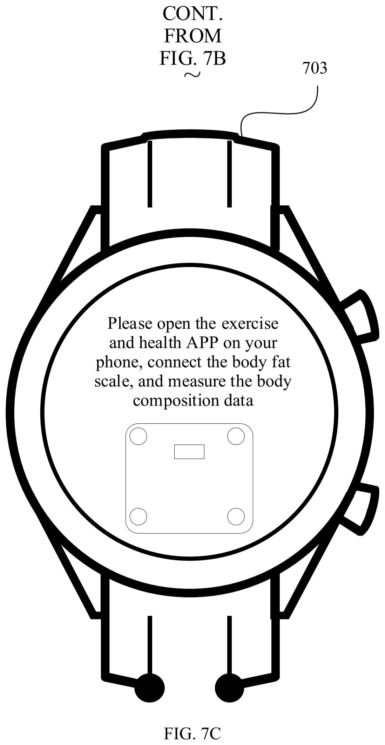

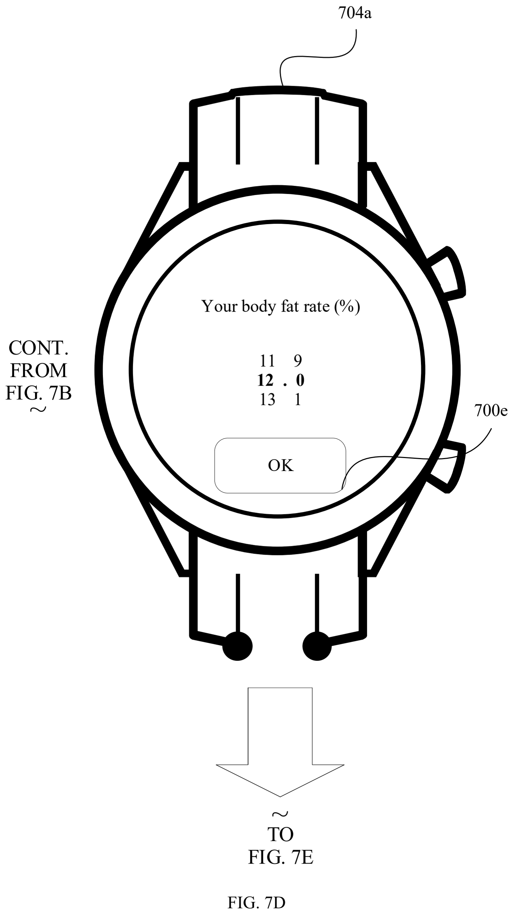

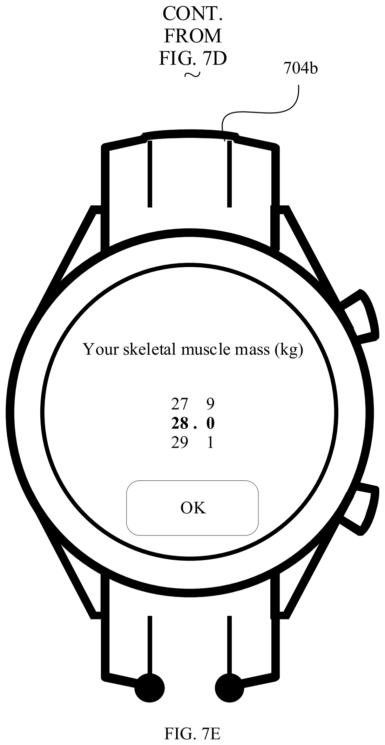

In another optional embodiment, body composition information may be obtained by using a body fat scale. The body composition information may include but is not limited to fat-free mass, fat mass, muscle mass, skeletal muscle mass, a water content, and a body fat rate. For example, FIG. 7A, FIG. 7B, FIG. 7C, FIG. 7D, and FIG. 7E are a diagram of interfaces for obtaining body composition information according to an embodiment of this application. An example in which obtaining body composition data is triggered on a smartwatch is used. The smartwatch may display an interface 702 in response to an operation performed on a control 700a on an interface 701. The interface 701 may be, for example, a display interface of an exercise and health application (application, APP) on the smartwatch, for checking some data related to exercise and health. For example, the control 700a is configured to check a resting metabolic rate. The interface 702 may be configured to guide the user to input the body composition data, including but not limited to: text prompt information for guiding the user to input the body composition data, a control 700b configured to guide the user to go to measure the body composition information, a control 700c configured to guide the user to manually enter the body composition information, and a control 700d configured to guide the user to skip entering the body composition information. For example, the smartwatch may display an interface 703 in response to an operation performed on the control 700b on the interface 702. The interface 703 may be configured to guide the user to connect the connected mobile phone to a body fat scale through the exercise and health APP, to measure the body composition data through the body fat scale. In another example, the smartwatch may display an interface 704a in response to an operation performed on the control 700c on the interface 702, where the interface 704a may be configured to guide the user to manually enter body fat rate information; and the smartwatch may display an interface 704b in response to an operation performed on an "OK" control 700e on the interface 704a, where the interface 704b may be configured to guide the user to manually enter skeletal muscle mass information. It may be understood that, for other body composition information that needs to be manually entered by the user, refer to the descriptions of the interface 704a and the interface 704b, and details are not described herein again. It should be noted that, when the smartwatch or the mobile phone is connected to the body fat scale and stores the latest body composition data (for example, today's body composition data), the body composition data can be obtained without the guidance process shown in FIG. 7A, FIG. 7B, FIG. 7C, FIG. 7D, and FIG. 7E.

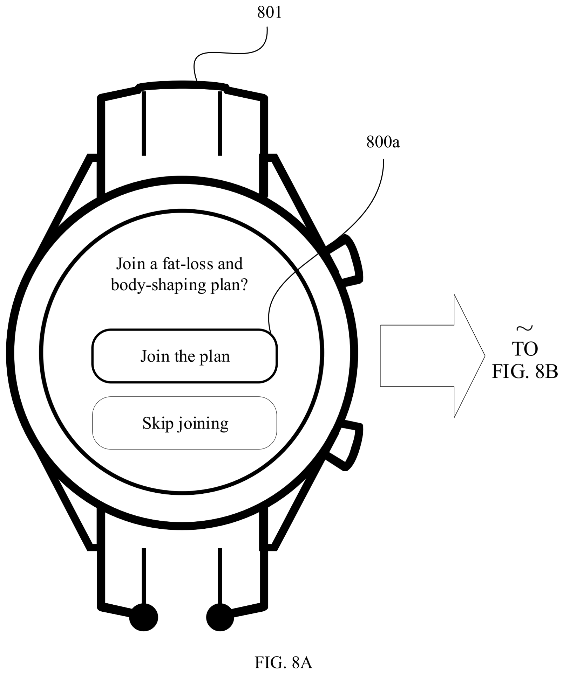

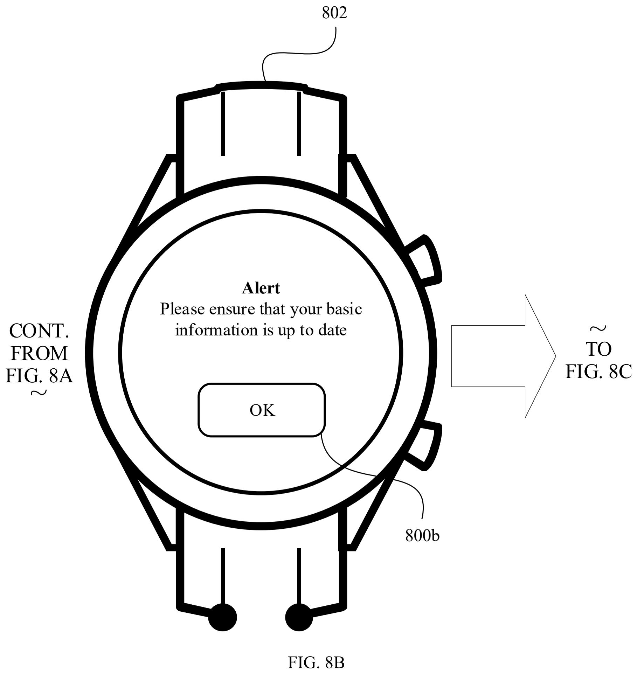

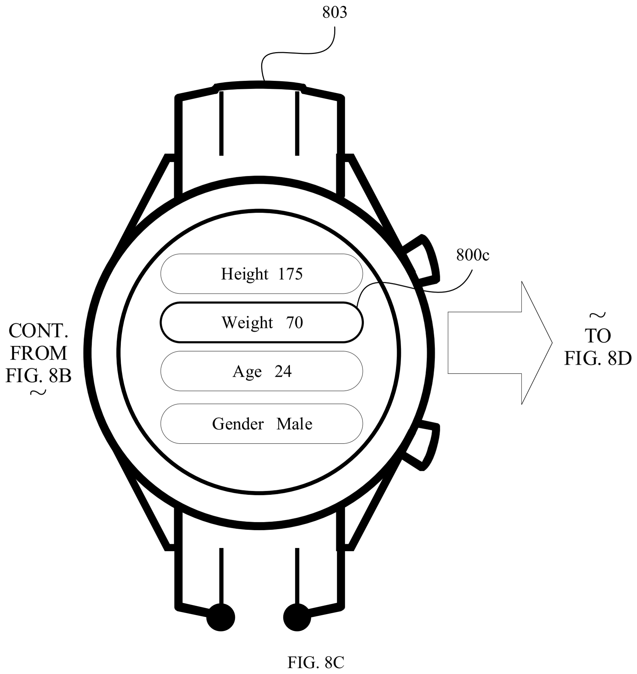

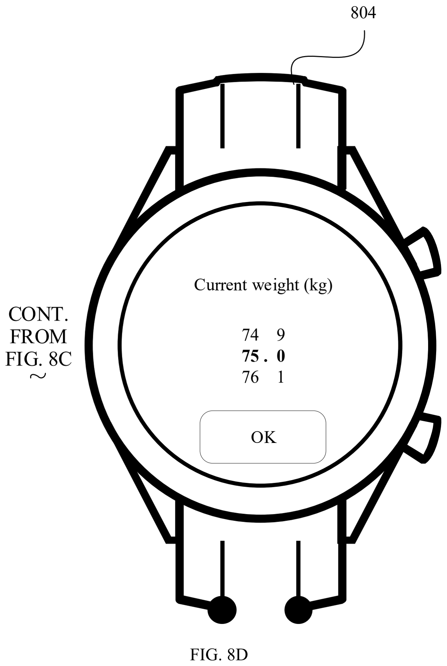

In addition, when basic information, such as weight, gender, height, and age, is not included in the terminal device or is not updated in a period of time, the terminal device may further obtain or update the basic information. For example, the mobile phone or the wearable device may obtain the basic information of the user in response to an operation input manually by the user. In another example, the mobile phone or the wearable device may further display a guidance interface to the user, to guide the user to input the basic information. For example, FIG. 8A, FIG. 8B, FIG. 8C, and FIG. 8D are another diagram of interfaces according to an embodiment of this application. An example in which obtaining basic information of the user is triggered on a smartwatch is used. The smartwatch may display an interface 802 in response to an operation performed on a control 800a on an interface 801. The interface 801 may be configured to indicate a prompt interface of a fat loss scenario, and the control 800a may be configured to indicate joining a fat-loss and body-shaping plan. It may be understood that the interface 801 may further include a control configured to indicate skipping joining a fat-loss and body-shaping plan. The interface 802 may be configured to prompt the user to determine the basic information to obtain the latest basic information, to generate a more accurate fat-loss and body-shaping plan based on the latest basic information. The smartwatch may display an interface 803 in response to an operation performed on an "OK" control 800b on the interface 802. The interface 803 may include a control configured to trigger input or update of height information, a control 800c configured to trigger input or update of weight information, a control configured to trigger input or update of age information, and a control configured to trigger input or update of gender information. For example, the smartwatch may display an interface 804 in response to an operation performed on the control 800c on the interface 803, and the interface 804 may be configured to input weight information. It should be noted that the basic information of the user may be further synchronized from another APP, for example, may be obtained from a weighing APP. In this case, manual input by the user as shown in FIG. 8A, FIG. 8B, FIG. 8C, and FIG. 8D is not required.

Step 602: Extract a parameter feature.

In an optional embodiment, the smartwatch may periodically collect physiological parameters such as a resting heart rate, respiration, a body temperature, a skin temperature, and a pressure, and may form a resting heart rate curve, a respiration curve, a body temperature curve, a skin temperature curve, a pressure curve, sleep quality, and the like after collecting a plurality of groups of physiological parameters within a period of time, to analyze and calculate time-domain features and/or frequency-domain features of the physiological parameters.

In another optional embodiment, through the calculation of the time-domain features and frequency-domain features, the smartwatch may obtain statistical features such as an average value, a standard deviation, a maximum value, a minimum value, and entropy of the physiological parameters in a period of time, and high-frequency and low-frequency component features in frequency domain. These features reflect physiological, psychological, and sleep statuses of the user in a period of time, and may affect a change of a resting metabolic rate. For example, a greater increase of an average value of the resting heart rate indicates a greater increase of the resting metabolic rate; a greater increase of an average body temperature value indicates a greater increase of the resting metabolic rate; larger fluctuation of the stress score indicates a more unstable resting metabolic rate; a stress score being higher than a stress threshold for a long time indicates a lower resting metabolic rate; and better sleep quality indicates a more stable resting metabolic rate.

In still another optional embodiment, the smartwatch may select, from the body composition data, a body composition feature highly related to the resting metabolic rate. For example, different body tissues exhibit varying energy expenditures. For example, energy expenditure of muscle is about three times that of fat. Therefore, the highly related body composition feature may include fat mass and muscle mass, and when the fat mass decreases and the muscle mass increases, the resting metabolic rate usually increases.

Step 603: Detect a resting metabolic rate.

In an optional embodiment, based on the parameter features in a period of time that are extracted in step 602, a machine learning regression model with fusion of multi-source parameters may be further established, to obtain hourly and/or daily resting metabolic rates in the period of time. In this way, a metabolic change trend in the period of time is analyzed, so that the user can be guided in time to adjust a status of the user.

Step 604: Display a user interaction interface based on the resting metabolic rate obtained through detection.

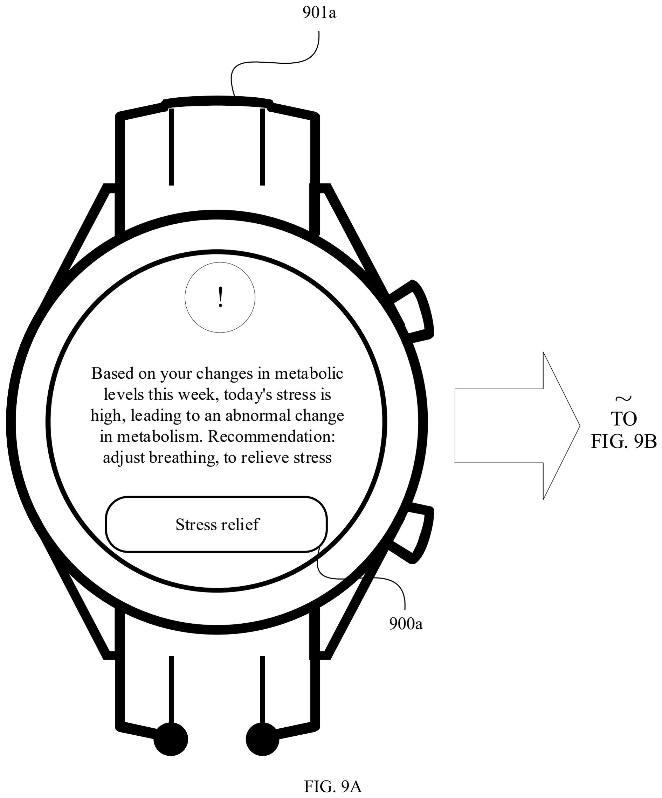

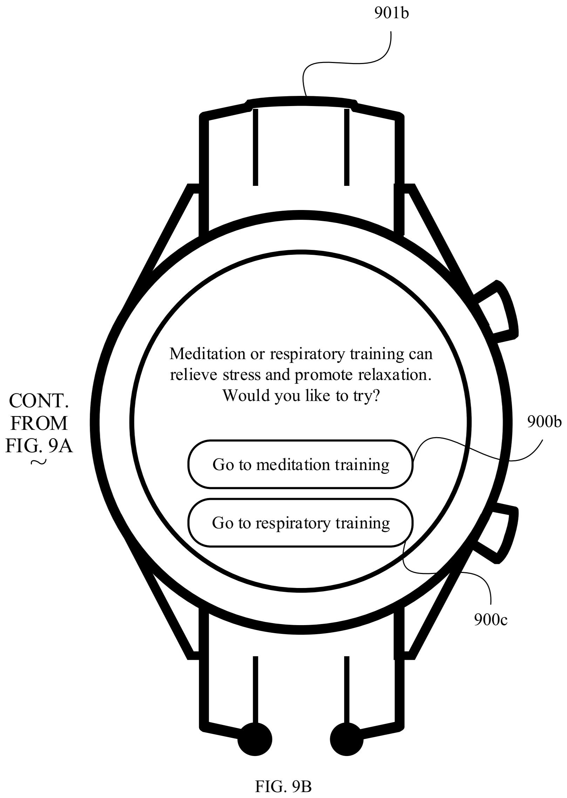

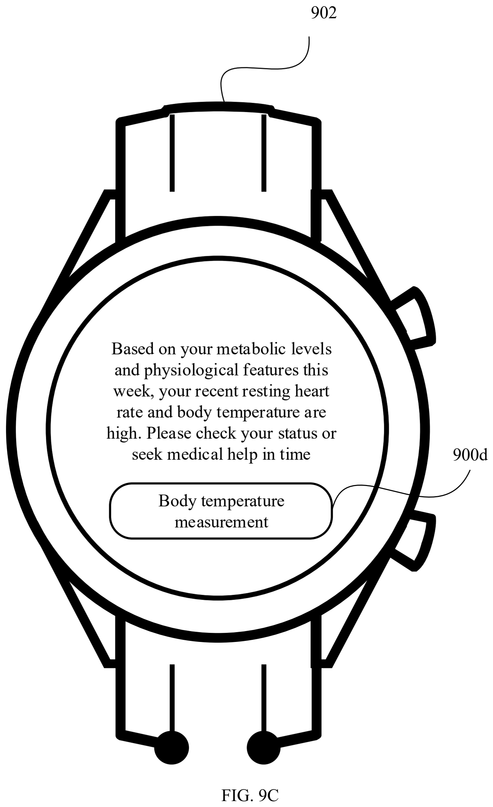

It may be understood that the resting metabolic rate is closely related to individual physiological, psychological, and sleep statuses. Through analysis on a metabolic change rate per day in a week and a metabolic change rate per hour in a day, a factor that causes an abnormal increase or decrease in metabolism is identified in time, the user is alerted to adjust physical and mental statuses or seek a medical examination in time, and the user is guided to maintain a good lifestyle in daily life and during weight loss or fat loss. For example, FIG. 9A, FIG. 9B, and FIG. 9C are a diagram of interfaces for a resting metabolic rate detection method according to an embodiment of this application. In a possible scenario, when the smartwatch detects an abnormal change in metabolism due to a factor such as high stress or insufficient sleep, the smartwatch may display an interface 901a to the user. The interface 901a may be configured to alert the user to an abnormal change of a resting metabolic rate and a cause of the abnormal change. The interface 901a may further include a "stress relief" control 900a configured to address the abnormal change. The smartwatch may display an interface 901b in response to an operation performed on the control 900a on the interface 901a. The interface 901b may be configured to alert the user to try meditation or respiratory training, to relieve anxiety and stress. The interface 901b may further include a "go to meditation training" control 900b and a "go to respiratory training" control 900c. In another possible scenario, when the smartwatch detects an abnormal increase in metabolism due to an increased body temperature or resting heart rate, the smartwatch may display an interface 902 to the user. The interface 902 may include text prompt information, and the interface 902 may further include a "body temperature measurement" control 900d. For example, the control 900d may be configured to trigger measurement timing of a thermometer.

According to the content described in the scenario B, in this embodiment, the physiological, psychological, and sleep parameters may be collected by using the wearable device such as a smartwatch or a smart band, and the body composition data may be further collected by using the body fat scale, so that change trends of daily and hourly resting metabolic rates of the user can be evaluated based on parameter features of collected multi-source parameters. In this way, timely alerts for abnormal decreases and increases of the resting metabolic rate can be made based on the change trend of the resting metabolic rate, so that the user can be guided to maintain a healthy lifestyle. Compared with determining a resting metabolic rate only based on information such as age, gender, height, and weight, where the determined resting metabolic rate is fixed, this scenario can implement detection of a change trend of the resting metabolic rate, and can further provide guidance for a physical status based on the change trend of the resting metabolic rate.

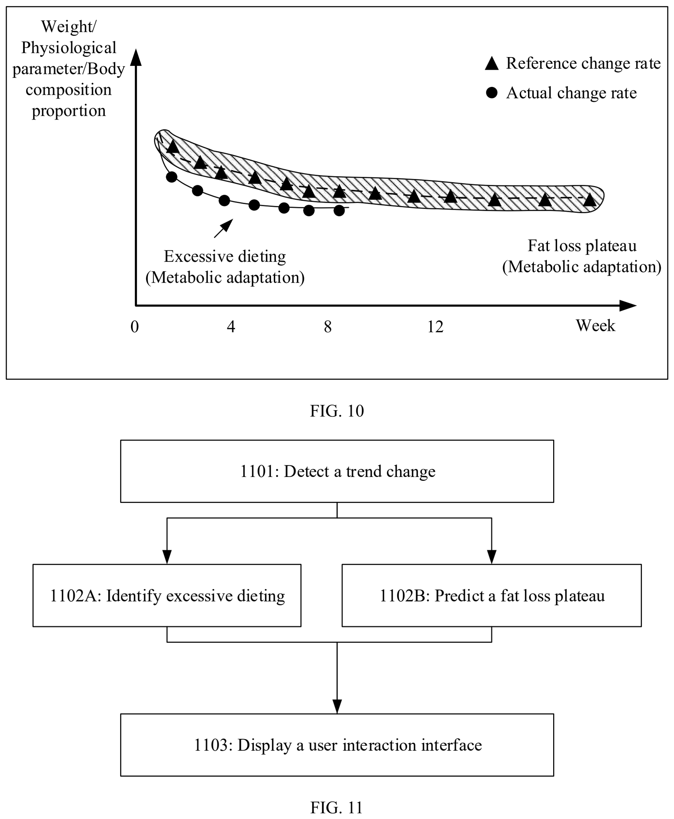

Scenario C: Fat loss scenario. During fat loss, metabolic adaptation may be identified based on a resting metabolic rate obtained through detection; whether a user is in an unhealthy dietary mode such as excessive dieting or starvation may be identified by assessing a decrease of the resting metabolic rate; and during long-term weight loss or fat loss, whether a fat loss plateau is entered may be further predicted, so that the user can be alerted in time to adjust an exercise plan and/or a dietary plan. For example, FIG. 10 is a diagram of a weight change rate according to an embodiment of this application. As shown in FIG. 10, a reference change rate denotes a change rate range and trend of a weight, a physiological parameter, or a body composition proportion during reasonable and scientific weight loss or fat loss. An actual change rate denotes a change rate and trend that are obtained through detection based on data collected by a body fat scale, a wearable device, and the like.

At an early stage of weight loss or fat loss, when the user quickly loses weight through excessive dieting, fasting, or starvation, physiological parameters such as a weight, a resting heart rate, and resting heart rate variability of the user change greatly, and a metabolic adaptation phenomenon occurs in advance, that is, the weight loss stalls after a period of time. This is a protective mechanism for the body to spontaneously reduce the resting metabolic rate and energy expenditure. For example, a change rate trend from week 0 to week 8 in FIG. 10 may be a metabolic adaptation phenomenon caused by excessive dieting, fasting, or starvation.

At a mid-to-late stage of weight loss or fat loss, after the weight decreases significantly, metabolic adaptation leads to a decrease in metabolism, and then a fat loss plateau may be entered. For example, a predicted reference change rate trend after week 12 in FIG. 10 may be a metabolic adaptation phenomenon caused by entering the fat loss plateau.

For example, FIG. 11 is a schematic flowchart of resting metabolic rate detection according to an embodiment of this application. The procedure may include the following steps.

Step 1101: Detect a trend change.

In a possible scenario, physiological data such as a resting heart rate and resting heart rate variability may be collected by using a wearable device such as a smartwatch or a smart band, and a change rate of the physiological data such as the resting heart rate or resting heart rate variability in a specific period of time is tracked and analyzed. For example, a change trend of the physiological data in one week, two weeks, or one month may be tracked.

In another possible scenario, weight and body composition data may be further collected by using a body fat scale or may be manually input by the user, and a weight loss rate in a specific period of time is tracked and analyzed. For example, a weight loss rate in one week may be tracked. In addition, in some possible scenarios, the mobile phone or the wearable device may further obtain diet of the user through manual recording or photo recording by the user, so that a change trend of caloric intake in a specific period of time can be further tracked and analyzed.

Step 1102A: Identify excessive dieting.

In an optional embodiment, when the mobile phone or the wearable device detects that changes of the weight, resting heart rate or resting heart rate variability, muscle mass, and body water content of the user in a preset period of time (for example, one month) meet preset conditions, an excessive dieting state may be determined. For example, the preset condition may include that: a weight decrease is greater than a weight threshold by x%, a change rate of an indicator such as a resting heart rate or resting heart rate variability is greater than a threshold y, and a decrease of muscle mass or body water content exceeds a threshold z.

Further, when the mobile phone or the wearable device detects an excessive dieting state, a decrease and an actual change rate may be further input to a pre-trained model, to obtain an additional decrease caused by metabolic adaptation. In this way, more accurate guidance can be provided for the user based on the additional decrease obtained through calculation, for example, a specific increase of calorie intake can be provided for the user.

In another optional embodiment, the excessive dieting state may be further identified with reference to a calorie intake change obtained based on recorded diet. For example, when it is detected that the calorie intake corresponding to the recorded diet is less than a calorie threshold K, the excessive dieting state may be determined.

Step 1102B: Predict a fat loss plateau.

In a possible embodiment, the mobile phone or the wearable device may track and analyze long-term change rates of weight and physiological indicators, and a long-term change of body composition (such as skeletal muscle mass or fat mass), to determine an actual decrease of a resting metabolic rate. For example, when the mobile phone or the wearable device detects that a weight decrease per month gradually decreases compared with a previous month, and an improvement in a physiological indicator (such as a resting heart rate or resting heart rate variability) gradually slows down, it may be predicted that the fat loss plateau is entered. For example, when a decrease is less than a threshold, it may be predicted that the fat loss plateau is entered.

Further, when the mobile phone or the wearable device presets that the fat loss plateau is entered, change rates of the foregoing parameters may be further input to a pre-trained model, to calculate an additional decrease caused by metabolic adaptation. In this way, the user can be alerted, based on the additional decrease obtained through calculation, to adjust an exercise plan, for example, appropriately increase exercise intensity or change an exercise type, thereby improving fat loss effect.

Step 1103: Display a user interaction interface.

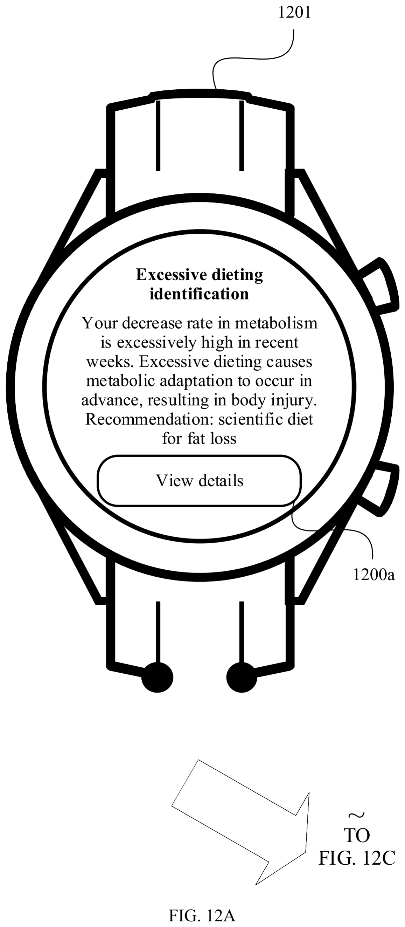

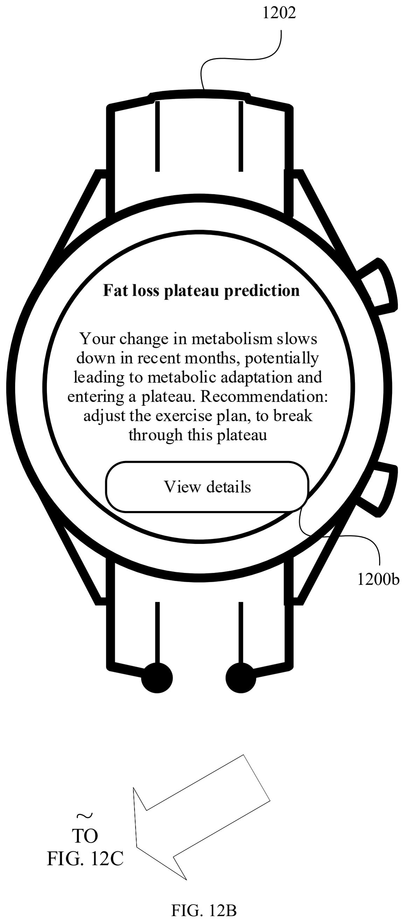

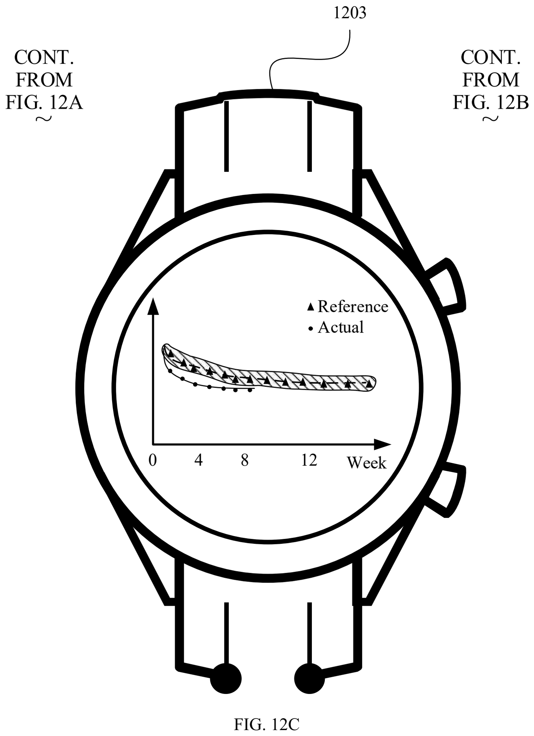

In an optional embodiment, the interaction interface may be displayed on the mobile phone or the smartwatch to the user in a form of a pop-up window, a notification bar, vibration, a ringtone, or the like, to guide the user to adjust a physical status. For example, FIG. 12A, FIG. 12B, and FIG. 12C are still another diagram of interfaces for a resting metabolic rate detection method according to an embodiment of this application. An example in which a user interaction interface is displayed on a smartwatch is used. In a possible scenario, when the smartwatch detects an excessive dieting state, an interface 1201 may be displayed. The interface 1201 may be configured to alert the user to a possibility of metabolic adaptation caused by the excessive dieting state, and provide a dietary recommendation. The interface 1201 may further include a "view details" control 1200a. In another possible scenario, when the smartwatch detects a fat loss plateau, an interface 1202 may be displayed. The interface 1202 may be configured to alert the user to a possibility of entering the fat loss plateau, and provide an exercise adjustment recommendation. The interface 1202 may further include a "view details" control 1200b. The smartwatch may display an interface 1203 in response to an operation performed on the control 1200a on the interface 1201 or in response to an operation performed on the control 1200b on the interface 1202. The interface 1203 may be configured to display a comparison between a reference change curve and an actual change curve, to facilitate a clearer and more visual understanding of the change.

According to the content described in the scenario C, in this embodiment, long-term change trends of physiological, weight, body composition, and diet records are tracked and analyzed. Metabolic adaptation is identified based on a degree of a rate change and a change trend of the resting metabolic rate. Therefore, an excessive dieting state can be detected, and a fat loss plateau can be predicted. In this way, healthier and more scientific diet and exercise guidance can be provided, thereby implementing scientific fat loss and avoiding body injury.

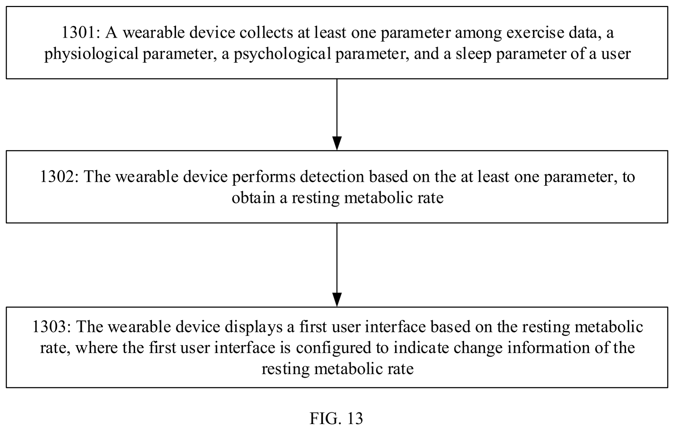

In another optional implementation, FIG. 13 is a schematic flowchart of a resting metabolic rate detection method according to an embodiment of this application. The procedure may include the following steps.

Step 1301: A wearable device collects at least one parameter among exercise data, a physiological parameter, a psychological parameter, and a sleep parameter of a user.

Step 1302: The wearable device performs detection based on the at least one parameter, to obtain a resting metabolic rate. For a detection manner of the wearable device, refer to the content described in the foregoing scenario A to scenario C. Details are not described herein again.

Step 1303: The wearable device displays a first user interface based on the resting metabolic rate, where the first user interface is configured to indicate change information of the resting metabolic rate. For the user interface, refer to the user interfaces in the foregoing scenario A to scenario C. Details are not described herein again.

Based on the foregoing embodiments, this application further provides a wearable device. The wearable device includes a plurality of functional modules. The plurality of functional modules interact with each other to implement functions performed by the wearable device in the methods described in embodiments of this application. For example, step 301 to step 306 performed by the wearable device in the embodiment shown in FIG. 3 may be performed, or step 601 to step 604 performed by the wearable device in the embodiment shown in FIG. 6 may be performed, or step 1101 to step 1103 performed by the wearable device in the embodiment shown in FIG. 11 may be performed, or step 1301 to step 1303 performed by the wearable device in the embodiment shown in FIG. 13 may be performed. The plurality of functional modules may be implemented based on software, hardware, or a combination of software and hardware, and the plurality of functional modules may be randomly combined or divided based on specific implementation.

Based on the foregoing embodiments, this application further provides a wearable device. The wearable device includes at least one processor and at least one memory. The at least one memory stores computer program instructions. When the wearable device runs, the at least one processor performs functions performed by the wearable device in the methods described in embodiments of this application. For example, step 301 to step 306 performed by the wearable device in the embodiment shown in FIG. 3 may be performed, or step 601 to step 604 performed by the wearable device in the embodiment shown in FIG. 6 may be performed, or step 1101 to step 1103 performed by the wearable device in the embodiment shown in FIG. 11 may be performed, or step 1301 to step 1303 performed by the wearable device in the embodiment shown in FIG. 13 may be performed. Based on the foregoing embodiments, this application further provides a resting metabolic rate detection system. The system includes the wearable device in the foregoing embodiments and a terminal device (for example, a terminal device with a processing capability, such as a mobile phone or a tablet computer). In a possible design, the system may further include the body fat scale in the foregoing embodiments.

Based on the foregoing embodiments, this application further provides a computer program product. The computer program product includes a computer program (which may also be referred to as code or instructions). When the computer program is run, a computer is enabled to perform the methods described in embodiments of this application.

Based on the foregoing embodiments, this application further provides a computer-readable storage medium. The computer-readable storage medium stores a computer program. When the computer program is executed by a computer, the computer is enabled to perform the methods described in embodiments of this application.

Based on the foregoing embodiments, this application further provides a chip. The chip is configured to read a computer program stored in a memory, to implement the methods described in embodiments of this application.

Based on the foregoing embodiments, this application provides a chip system. The chip system includes a processor, configured to support a computer apparatus in implementing the methods described in embodiments of this application. In a possible design, the chip system further includes a memory, and the memory is configured to store a program and data that are necessary for the computer apparatus. The chip system may include a chip, or may include a chip and another discrete component.

A person skilled in the art should understand that embodiments of this application may be provided as a method, a system, or a computer program product. Therefore, this application may use a form of hardware only embodiments, software only embodiments, or embodiments with a combination of software and hardware. In addition, this application may use a form of a computer program product that is implemented on one or more computer-usable storage media (including but not limited to a magnetic disk memory, a CD-ROM, and an optical memory) that include computer-usable program code.

This application is described with reference to the flowcharts and/or block diagrams of the method, the device (system), and the computer program product according to this application. It should be understood that computer program instructions may be used to implement each process and/or each block in the flowcharts and/or the block diagrams and a combination of a process and/or a block in the flowcharts and/or the block diagrams. These computer program instructions may be provided for a general-purpose computer, a dedicated computer, an embedded processor, or a processor of another programmable data processing device to generate a machine, so that the instructions executed by a computer or a processor of another programmable data processing device generate an apparatus for implementing a specific function in one or more processes in the flowcharts and/or in one or more blocks in the block diagrams.

These computer program instructions may alternatively be stored in a computer-readable memory that can instruct the computer or the another programmable data processing device to work in a specific manner, so that instructions stored in the computer-readable memory generate an artifact that includes an instruction apparatus. The instruction apparatus implements a function specified in one or more processes in the flowcharts and/or in one or more blocks in the block diagrams.

These computer program instructions may alternatively be loaded onto the computer or the another programmable data processing device, so that a series of operations and steps are performed on the computer or the another programmable device, to generate computer-implemented processing. Therefore, the instructions executed on the computer or the another programmable device provide steps for implementing a function specified in one or more processes in the flowcharts and/or in one or more blocks in the block diagrams.

It is clear that a person skilled in the art can make various modifications and variations to this application without departing from the protection scope of this application. This application is intended to cover these modifications and variations of this application provided that they fall within the scope of the claims of this application and their equivalent technologies.

## Claims

1. A resting metabolic rate detection method, comprising:
collecting, by a wearable device, at least one parameter among exercise data, a physiological parameter, a psychological parameter, and a sleep parameter of a user;
performing, by the wearable device, detection based on the at least one parameter, to obtain a resting metabolic rate; and
displaying, by the wearable device, a first user interface based on the resting metabolic rate, wherein the first user interface is configured to indicate change information of the resting metabolic rate.

2. The method according to claim 1, wherein the wearable device collects the exercise data of the user; and
performing, by the wearable device, detection based on the exercise data, to obtain the resting metabolic rate comprises:
determining exercise duration and an average heart rate during exercise based on the exercise data;
determining, based on the exercise duration, the average heart rate, and a preset matching relationship, exercise intensity that matches the exercise data, wherein the preset matching relationship indicates an exercise duration range and an average heart rate range that match different exercise intensity; and
determining the resting metabolic rate based on the exercise intensity and the exercise duration, wherein the resting metabolic rate indicates a resting metabolic rate after the exercise.

3. The method according to claim 2, wherein determining the resting metabolic rate based on the exercise intensity and the exercise duration comprises:
determining incremental information of the resting metabolic rate by using a pre-trained multi-parameter regression model with the exercise intensity and the exercise duration as inputs; and
determining the resting metabolic rate after the exercise based on a resting metabolic rate before the exercise and the incremental information.

4. The method according to any one of claims 1 to 3, wherein the exercise data comprises at least one of a photoplethysmography PPG signal, an accelerometer ACC signal, and an inertial measurement unit IMU.

5. The method according to any one of claims 1 to 4, wherein the first user interface comprises an hourly, daily, weekly, monthly, or yearly resting metabolic rate and change information thereof, wherein
in a case that the first user interface comprises the hourly resting metabolic rate, when the wearable device collects the exercise data, the resting metabolic rate is higher than a resting metabolic rate obtained before the exercise data is collected.

6. The method according to any one of claims 1 to 4, wherein the first user interface comprises at least one of daily calorie expenditure, daily calorie intake, and a daily calorie deficit, and the daily calorie deficit is determined based on a difference between the daily calorie expenditure and the daily calorie intake.

7. The method according to any one of claims 1 to 6, wherein before performing detection to obtain the resting metabolic rate, the method further comprises:
displaying, by the wearable device, a second user interface, wherein the second user interface is configured to guide the user to input body composition information, and the second user interface comprises a first control and a second control; and
obtaining, by the wearable device in response to a first operation on the first control, the body composition information measured by a body fat scale; or
displaying, by the wearable device in response to a second operation on the second control, at least one input interface, wherein the at least one input interface is configured to manually input at least one body composition parameter comprised in the body composition information; and
performing, by the wearable device, detection based on the at least one parameter, to obtain the resting metabolic rate comprises:
performing, by the wearable device, detection based on the at least one parameter and the body composition parameter, to obtain the resting metabolic rate.

8. The method according to any one of claims 1 to 7, wherein after performing detection to obtain the resting metabolic rate, the method further comprises:
displaying a third user interface based on the resting metabolic rate and a historical resting metabolic rate when a decrease of the resting metabolic rate meets a preset condition, wherein the third user interface is configured to alert the user to the decrease of the resting metabolic rate.

9. The method according to claim 8, wherein the wearable device stores daily calorie intake of the user, and when the daily calorie intake is less than a calorie intake threshold, the third user interface is further configured to make an alert for an excessive dieting state.

10. The method according to claim 8 or 9, wherein when the wearable device detects that there is an ongoing fat loss plan, the third user interface is further configured to make an alert for a fat loss plateau.

11. The method according to any one of claims 1 to 10, wherein after performing detection to obtain the resting metabolic rate, the method further comprises:
sending the resting metabolic rate to a terminal device, wherein the terminal device is configured to display a fourth user interface based on the resting metabolic rate, and the fourth user interface is configured to indicate the change information of the resting metabolic rate.

12. The method according to any one of claims 1 to 11, wherein the physiological parameter comprises at least one of the following parameters: a resting heart rate, resting heart rate variability, a body temperature, a skin temperature, and a respiratory rate.

13. The method according to any one of claims 1 to 12, wherein the psychological parameter comprises at least one of the following parameters: a stress score and a stress level.

14. The method according to any one of claims 1 to 13, wherein the sleep parameter comprises at least one of the following parameters: sleep time, sleep duration, sleep quality, and deep sleep continuity.

15. The method according to any one of claims 1 to 14, wherein displaying, by the wearable device, the first user interface based on the resting metabolic rate comprises:
displaying, by the wearable device, the first user interface based on the resting metabolic rate and the at least one parameter, wherein the first user interface is further configured to indicate a parameter that affects the change information of the resting metabolic rate.

16. A wearable device, comprising one or more processors and one or more memories, wherein
the one or more memories are configured to store one or more computer programs and data information, wherein the one or more computer programs comprise instructions; and
when the instructions are executed by the one or more processors, the wearable device is enabled to perform the method according to any one of claims 1 to 15.

17. A resting metabolic rate detection system, comprising the wearable device according to claim 16 and a terminal device.

18. The system according to claim 17, further comprising a body fat scale.

19. A computer-readable storage medium, wherein the computer-readable storage medium is configured to store a computer program, and when the computer program is run on a computer, the computer is enabled to perform the method according to any one of claims 1 to 15.

20. A computer program product, comprising a computer program, wherein when the computer program is run on a computer, the computer is enabled to perform the method according to any one of claims 1 to 15.
